# EUROPEAN PATENT APPLICATION

(11) **EP 4 434 586 A1**
(43) Date of publication of application: **25.09.2024**
(21) Application number: 23305391.7
(22) Date of filing: 23.03.2023
(51) Int. Cl.: A61P 35/00, A61P 35/02, C07K 16/18, C07K 16/28, A61K 39/00

(54) **COMBINATION OF ANTIBODIES, OR ANTIGEN-BINDING FRAGMENTS THEREOF, FOR THE TREATMENT OF CANCER AND COMPOSITION THEREOF**

(71) Applicant: Xenothera, 44200 Nantes (FR)
(72) Inventor: The designation of the inventor has not yet been filed
(74) Representative: Cabinet Nony

(57) **Abstract**

The present invention relates to a combination of antibodies, or antigen-binding fragments thereof, for use in the treatment of a cancer in a human subject in need thereof, the combination comprising at least a first antibody, or an antigen-binding fragment thereof, at least a second antibody, or an antigen-binding fragment thereof, and at least a third antibody, or an antigen-binding fragment thereof, each of these antibodies independently and specifically binding to an antigen selected from the group consisting of SLC3A2, CKAP4, Annexin A2, GSTO1, Annexin A5, MX1, RTN4, FASN and PDIA4.

The present invention further relates to a pharmaceutical composition for use in the treatment of a cancer in a human subject in need thereof comprising this combination of antibodies, or antigen-binding fragments thereof and a pharmaceutically acceptable carrier.

## Description

### Field of the Invention

The present invention relates to the field of treatment of cancers. In particular, the present invention relates to the field of combinations of antibodies directed against specific antigens and their implementation in treatment of cancers.

### Description of Related Art

Cancer is the second leading cause of death in the World and is responsible for an estimated 9.6 million deaths in 2018. Globally, about 1 in 6 deaths worldwide is due to cancer. Inaccessible or non-sufficiently active treatment are common in this filed. There is thus a well-recognized need to develop new and improved therapies for treating cancers.

Immunotherapies represent a real hope in the treatment of a great number of cancers, in particular in the treatment of cancers not currently efficiently cured by conventional therapies. This treatment mainly consists in the administration of monoclonal antibodies directed against the tumoral cells or directed against activators or inhibitors of a checkpoint of the immune response against cancers.

Antibodies directed against tumors (passive immunotherapy) can act through two complementary ways:
- by cytotoxicity complement dependent (CDC) or dependent from killer cells (ADCC) or dependent from phagocytes (ADCP).
- by induction of an adaptative immune response. It has indeed been demonstrated that opsonization of a target and the local production of complement's molecules (C3a, C5a) (see Strainic et al., Immunity, 2008 Mar;28(3):425-35) activates the T lymphocyte co-stimulation and increases its survival. Thus, the passive administration of antitumor antibodies can, as a first step, generate the discharge of factors from the complement that, in a second step, ease the T cells response.

However, 40% to 85% of the patients are resistant to treatments based on the use of monoclonal antibodies. The escaping mechanisms comprise immunoselection mechanisms, i.e. the capacity of the tumoral cell to lose the antigen recognized by the immune system and immunosubversion mechanisms (induction of a specific tolerance). The appearance of less immunogenic tumoral variants can be particularly deleterious within treatments based on the use of monoclonal antibodies (which are specific to a unique epitope).

Accordingly, a treatment based on the use of combinations of antibodies targeting different antigens on the tumoral cells might allow minimizing the escaping mechanisms.

However, despite their potential efficacy as observed by Richet more than 100 years ago, combinations of antibodies, such as polyclonal antibodies of animal origin, have rarely been used in the treatment of tumors due in particular to high toxic risks in patients. This toxicity is mainly linked to the expression of Neu5GC and alpha-1,3-galactose carbohydrates on animal immunoglobulins which in human elicit potent anti-Neu5GC and anti-alpha-1,3-galactose immune responses associated with allergy, serum sickness disease and formation of immune complexes. In order to mimic the polyclonal humoral immune response for cancer treatment, combinations of monoclonal have been considered. However, their clinical efficacy as monotherapy does not predicate their safety and clinical efficacy in combination (Berlin et al., Investigational News Drugs (2022) 40:586-595). The difficulty of such combination mainly lies in the association of antibodies with different PKs (which makes it more difficult to control toxicity), the stability of the product according to the isotypes and the manufacturing (see for example Larbouret et al., Cancers (Basel). 2021 Sep 15;13(18):4620).

There accordingly remains a need in the field for novel anti-cancer therapeutic agents, and in particular novel therapeutic agents efficient against a great variety of cancers.

There also remains a need in the art for the provision of novel anti-cancer therapeutic agents able to bind to tumoral cells, to activate complement and to activate apoptotic mechanisms to kill a great variety of cancers.

There also remains a need in the art for the provision of an effective and improved therapy for treating cancers and endowed with reduced or no adverse effects.

The invention has for purpose to meet the above-mentioned needs.

### Summary of the invention

The present invention in particular relates to the following items:
**Item 1:** a combination of antibodies, or antigen-binding fragments thereof, for use in the treatment of a cancer in a human subject in need thereof, the combination comprising:
   - at least a first antibody, or an antigen-binding fragment thereof,
   - at least a second antibody, or an antigen-binding fragment thereof, and
   - at least a third antibody, or an antigen-binding fragment thereof,

   each of these antibodies independently and specifically binding to an antigen selected from the group consisting of SLC3A2, CKAP4, Annexin A2, GSTO1, Annexin A5, MX1, RTN4, FASN and PDIA4,
   wherein the first, second and third antibodies, or antigen-binding fragments thereof, each bind to a different antigen selected from the group consisting of SLC3A2, CKAP4, Annexin A2, GSTO1, Annexin A5, MX1, RTN4, FASN and PDIA4.
   In some interesting and unexpected way, the inventors have indeed determined, and demonstrated as illustrated in the examples, the ability of various combinations of antibodies, and in particular of non-human mammal polyclonal antibodies, directed against specific antigens to strongly and specifically target, with no apparent toxicity, a variety of cancers, though these cancers are not derived from same tissues.
   These findings clearly go against the well-established prejudice according to which combinations of more than two antibodies cannot be implemented in the treatment of cancers considering their expected high toxic effect, as explained above, in the patient to be treated.
   It moreover interestingly provides clinicians new active agents that can be implemented against a wide variety of cancers.
**Item 2:** the combination for use according to item 1, comprising at least a fourth antibody, or an antigen binding fragment thereof, specifically binding to an antigen selected from the group consisting of SLC3A2, CKAP4, Annexin A2, GSTO1, Annexin A5, MX1, RTN4, FASN and PDIA4,
   said antigen being in particular different from the three antigens to which the first, second and third antibodies, or antigen-binding fragments thereof, bind to.
**Item 3:** the combination for use according to claim 1 or 2, wherein the antigen-binding fragments of the antibodies present in the combination are independently Fv, Fab, F(ab')2, Fab', dsFv, (dsFv)2, scFv, sc(Fv)2, or diabodies.
**Item 4:** the combination for use according to any one of items 1 to 3, wherein the cancer is selected from the group consisting of myeloma; melanoma; breast cancer; prostate cancer; colorectal cancer, in particular a colon cancer; lung cancer, in particular non small cell lung cancer; liver cancer, in particular an hepatocarcinoma; pancreas cancer; lymphoma, in particular B-cell or T-cell lymphoma, more particularly T-cell lymphoma; gastric cancer; head and neck cancer; ovarian cancer; sarcoma; leukemia, osteosarcoma; anal cancer; testicular cancer; uterus cancer; thyroid cancer; cancer of the central nervous system; gastrointestinal stromal cancer; epidermal carcinoma and oesophageal cancer.
**Item 5:** the combination for use according to any one of claims 1 to 4, wherein:
   - the first antibody, or an antigen-binding fragment thereof, specifically binds to antigen GSTO1;
   - the second antibody, or an antigen-binding fragment thereof, specifically binds to antigen Annexin A5;
   - the third antibody, or an antigen-binding fragment thereof, specifically binds to antigen MX1; and
   - the fourth antibody, or an antigen-binding fragment thereof, specifically binds to antigen RTN4;
   - the cancer being selected from the group consisting of melanoma; myeloma; a liver cancer, in particular an hepatocarcinoma; breast cancer; prostate cancer; colorectal cancer, in particular a colon cancer; and lung cancer, in particular non small cell lung cancer;

   more particularly selected from the group consisting of melanoma; prostate cancer; myeloma; a liver cancer, in particular an hepatocarcinoma; colorectal cancer, in particular a colon cancer and lung cancer, in particular non small cell lung cancer;
   in particular selected from the group consisting of melanoma; prostate cancer; myeloma; a liver cancer, in particular an hepatocarcinoma and lung cancer, in particular non small cell lung cancer; and
   more particularly selected from the group consisting of melanoma; myeloma and a liver cancer, in particular an hepatocarcinoma.
**Item 6:** the combination for use according to any one of items 1 to 4, wherein:
   - the first antibody, or an antigen-binding fragment thereof, specifically binds to antigen Annexin A2;
   - the second antibody, or an antigen-binding fragment thereof, specifically binds to antigen SLC3A2;
   - the third antibody, or an antigen-binding fragment thereof, specifically binds to antigen CKAP4; and
   - the fourth antibody, or an antigen-binding fragment thereof, specifically binds to antigen PDIA4;
   - the cancer being selected from the group consisting of myeloma; melanoma; breast cancer; prostate cancer; a liver cancer, in particular an hepatocarcinoma; colorectal cancer, in particular a colon cancer; lung cancer, in particular non small cell lung cancer; pancreas cancer; lymphoma, in particular B-cell or T-cell lymphoma, more particularly T-cell lymphoma; ovarian cancer; sarcoma; leukemia; osteosarcoma; testicular cancer; uterus cancer; thyroid cancer; cancer of the central nervous system; gastrointestinal stromal cancer and epidermal carcinoma;

   more particularly selected from the group consisting of myeloma; melanoma; breast cancer; prostate cancer; a liver cancer, in particular an hepatocarcinoma; colorectal cancer, in particular a colon cancer; lung cancer, in particular non small cell lung cancer; pancreas cancer and lymphoma, in particular T-cell lymphoma;
   even more particularly selected from the group consisting of myeloma; melanoma; breast cancer; a liver cancer, in particular an hepatocarcinoma; colorectal cancer, in particular a colon cancer; lung cancer, in particular non small cell lung cancer; pancreas cancer and lymphoma, in particular T-cell lymphoma;
   more particularly selected from the group consisting of myeloma; melanoma; a liver cancer, in particular an hepatocarcinoma and colorectal cancer, in particular a colon cancer; and
   in particular selected from the group consisting of myeloma; melanoma; and a liver cancer, in particular an hepatocarcinoma.
**Item** 7: the combination for use according to any one of items 1 to 4, wherein:
   - the first antibody, or an antigen-binding fragment thereof, specifically binds to antigen SLC3A2;
   - the second antibody, or an antigen-binding fragment thereof, specifically binds to antigen Annexin A5;
   - the third antibody, or an antigen-binding fragment thereof, specifically binds to antigen RTN4; and
   - the fourth antibody, or an antigen-binding fragment thereof, specifically binds to antigen FASN;
      wherein the combination further comprises a fifth antibody, or an antigen-binding fragment thereof, which specifically binds to antigen PDIA4;
   - the cancer being selected from the group consisting of a liver cancer, in particular an hepatocarcinoma; myeloma; melanoma; colorectal cancer, in particular a colon cancer; lung cancer, in particular non small cell lung cancer; gastric cancer; head and neck cancer; anal cancer and oesophageal cancer,

   in particular is selected from the group consisting of a liver cancer, in particular an hepatocarcinoma, myeloma; melanoma and a colorectal cancer, in particular a colon cancer;
   and is more particularly a liver cancer, in particular an hepatocarcinoma.
**Item 8:** the combination for use according to any one of items 1 to 7, wherein at least one of the antibodies of the combination for use, and in particular all the antibodies of the combination for use, is/are devoid of at least one antigenic determinant selected from (i) N-glycolylneuraminic acid (Neu5Gc) and (ii) α-1,3-galactose, and in particular is/are devoid of the two antigenic determinants N-glycolylneuraminic acid (Neu5Gc) and α-1,3-galactose.
**Item 9:** a pharmaceutical composition for use in the treatment of a cancer in a human subject in need thereof comprising a combination of antibodies, or antigen-binding fragments thereof, as defined in anyone of items 1 to 3 and 5 to 8, and a pharmaceutically acceptable carrier.
**Item 10:** the pharmaceutical composition for use according to item 9, wherein the cancer is selected from the group consisting of myeloma; melanoma; breast cancer; prostate cancer; colorectal cancer, in particular a colon cancer; lung cancer, in particular non small cell lung cancer; liver cancer, in particular an hepatocarcinoma; pancreas cancer; lymphoma, in particular B-cell or T-cell lymphoma, more particularly T-cell lymphoma; gastric cancer; head and neck cancer; ovarian cancer; sarcoma; leukemia, osteosarcoma; anal cancer; testicular cancer; uterus cancer; thyroid cancer; cancer of the central nervous system; gastrointestinal stromal cancer; epidermal carcinoma and oesophageal cancer.
**Item 11:** the pharmaceutical composition for use according to item 9 or 10, wherein the composition further comprises at least one additional anticancer drug different from the antibodies of the combination of antibodies as defined in anyone of claims 1 to 3 and 5 to 8.
**Item 12:** the polyclonal antibody composition for its use according to claim 11, wherein the at least one additional anticancer drug is selected from the group consisting of monoclonal antibodies, in particular selected from the group consisting of anti-CD137, anti-CTLA4, anti-TIM-3, anti-B7-H3, anti-CD134, anti-CD154, anti-LAG-3, anti-CD227, anti-BTNA3, anti-CD39, anti-CD73, anti-CD115, anti-CD47, anti-SIRP alpha, anti-SIRP gamma, anti-CD28, anti-NCR, anti-NKp46, anti-NKp30, anti-NKp44, anti-NKG2D, anti-PD1, anti-PDL1 and anti-DNAM-1 monoclonal antibodies.

### Brief description of the figures

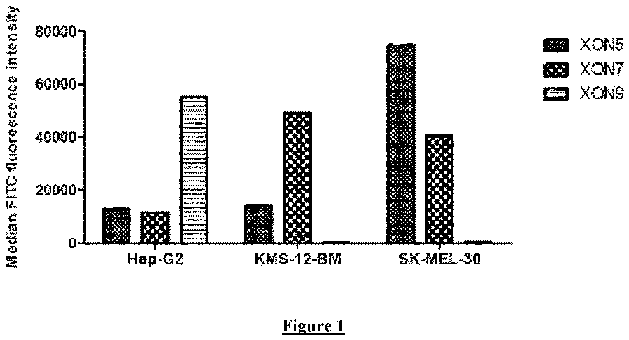

**Figure 1****:** describes cancer cell lines cross-reactivity assays by FACS (BD FACSCanto^{™}; BD Biosciences, US) of XON5, XON 7 or XON 9 pAbs on 100 000 cells from different human tumor cell lines. XON5 is a combination of anti-GSTO1, anti-Annexin 5, anti-MX1 and anti-RTN4 polyclonal antibodies obtained from a pig. XON7 is a combination of anti- Annexin A2, anti- SLC3A2, anti-CKAP4 and anti-PDIA4 polyclonal antibodies obtained from a pig. XON9 is a combination of anti-SLC3A2, anti-Annexin 5, anti-RTN4, anti-FASN and anti-PDIA4 polyclonal antibodies obtained from a pig.

A FITC anti-pig IgG was used as secondary antibody.

The human cell lines tested were HepG2 (human hepatocellular carcinoma), KMS-12-BM (human myeloma) and SK-MEL-30 (human melanoma).

Abscissa: the different human tumor cell lines. From left to right: Hep-G2, KMS-12-BM and SK-MEL-30.

Ordinate: Median FITC fluorescence intensity.

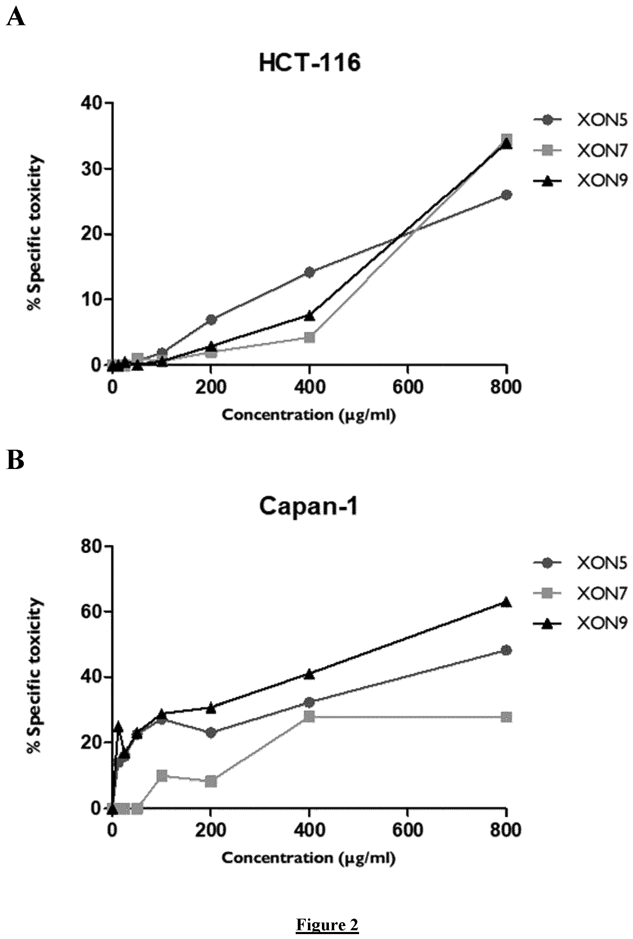

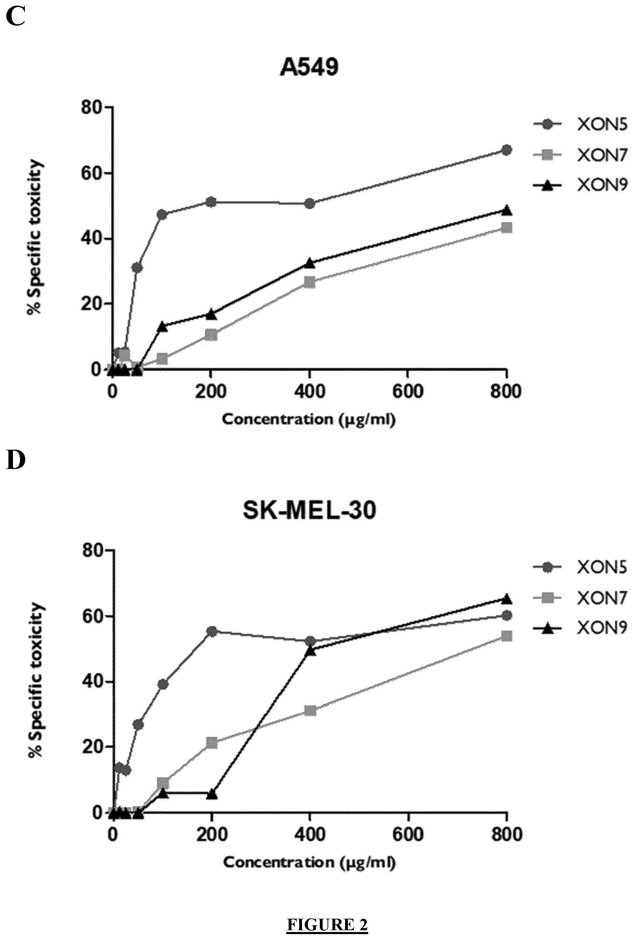

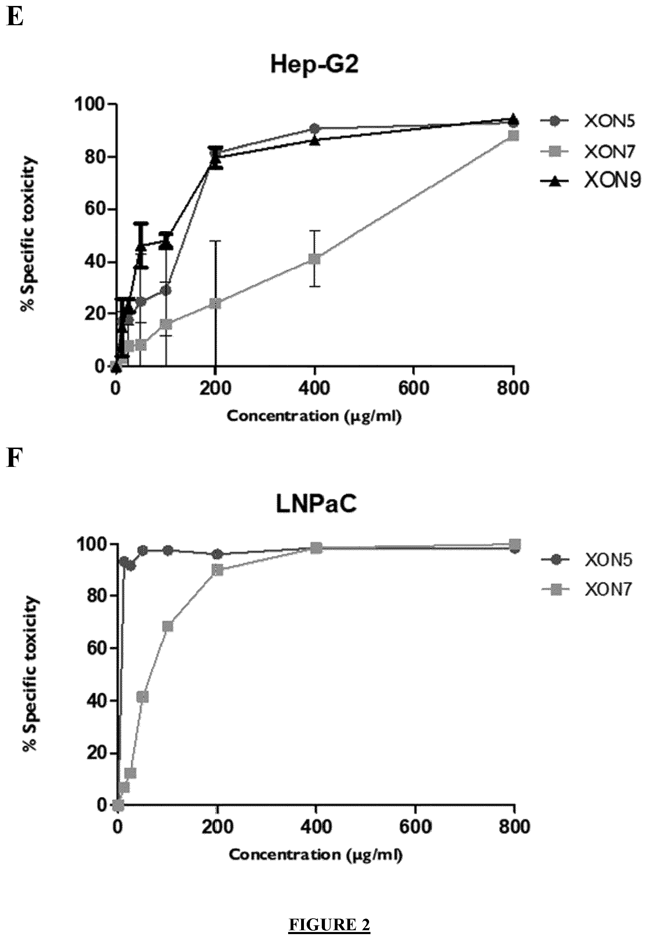

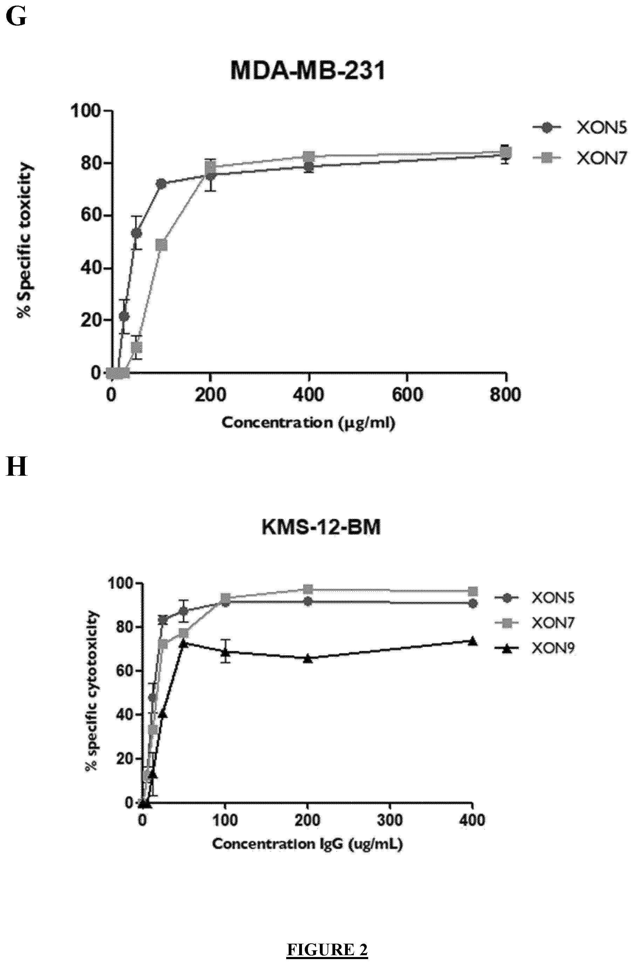

**Figure 2****:** describes the cross CDC activity of XON5, XON7 and XON9 as defined above against different human cancer cell lines.

Percentage of the specific cytotoxic activity of the different antibody combinations according to the invention XON5 ( 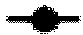 ); XON7 ( 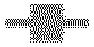 ) and XON9 ( 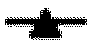 ).
Fig 2A/ XON5 ( 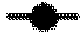 ); XON7 ( 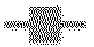 ) and XON9 ( 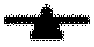 ) on HCT-116 cell line.
Fig 2B/ XON5 ( 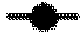 ); XON7 ( 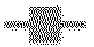 ) and XON9 ( 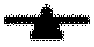 ) on Capan-1 cell line.
Fig 2C/ XON5 ( 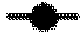 ); XON7 ( 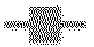 ) and XON9 ( 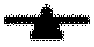 ) on A549 cell line.
Fig 2D/ XON5 ( 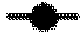 ); XON7 ( 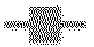 ) and XON9 ( 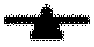 ) on SK-MEL-30 cell line.
Fig 2E/ XON5 ( 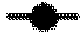 ); XON7 ( 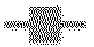 ) and XON9 ( 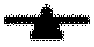 ) on Hep-G2 cell line.
Fig 2F/XON5 ( 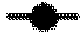 ) and XON7 ( 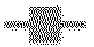 ) on LNPaC cell line.
Fig 2G/ XON5 ( 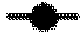 ) and XON7 ( 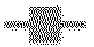 ) on MDA-MB-231 cell line.
Fig 2H/ XON5 ( 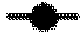 ); XON7 ( 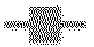 ) and XON9 ( 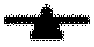 ) on KMS-12-BM cell line.

In each Figure 2A to 2H: ordinate: % of specific cytotoxicity; Abscissa: Concentration of the tested combinations of antibodies (µg/mL).

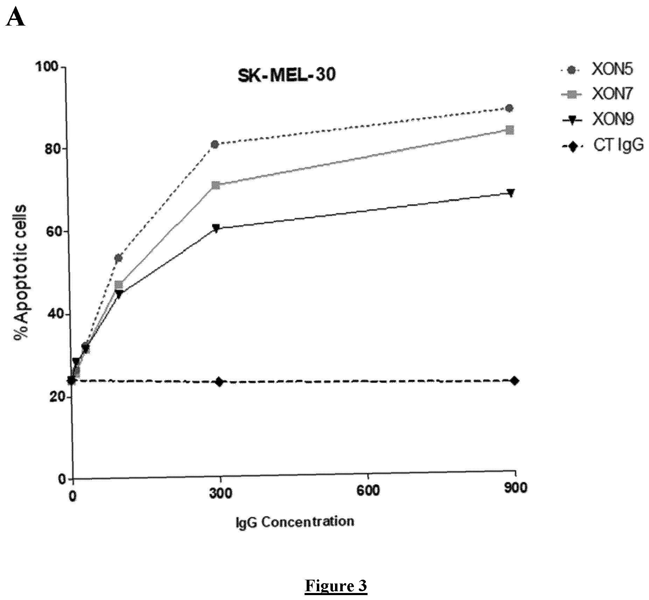

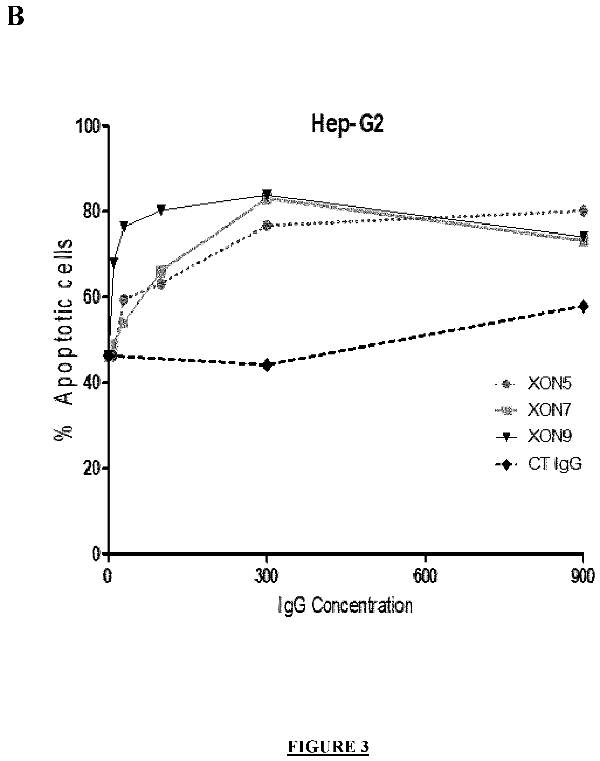

**Figure 3****:** describes cross apoptosis activity of XON5, XON7 and XON9 as defined above against different human cancer cell lines.

Percentage of the apoptotic cells (SK MEL-30 (Fig 3A) or HepG2 (Fig 3B)) after treatment with different antibody combinations according to the invention: XON5 ( 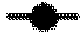 ); XON7 ( 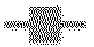 ), XON9 ( 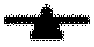 ) and Control IgG (CT IgG) (◆).

Fig 3A/ XON5 ( 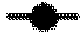 ); XON7 ( 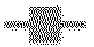 ), XON9 ( 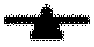 ) and Control IgG (◆) on SK MEL-30 cell line.

Fig 3B1 XON5 ( 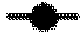 ); XON7 ( 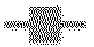 ); XON9 ( 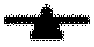 ) and Control IgG (◆) on HepG2 cell line.

In each Figure 3A and 3B: ordinate: % of apoptotic cells; Abscissa: Concentration of the tested antibodies (µg/mL).

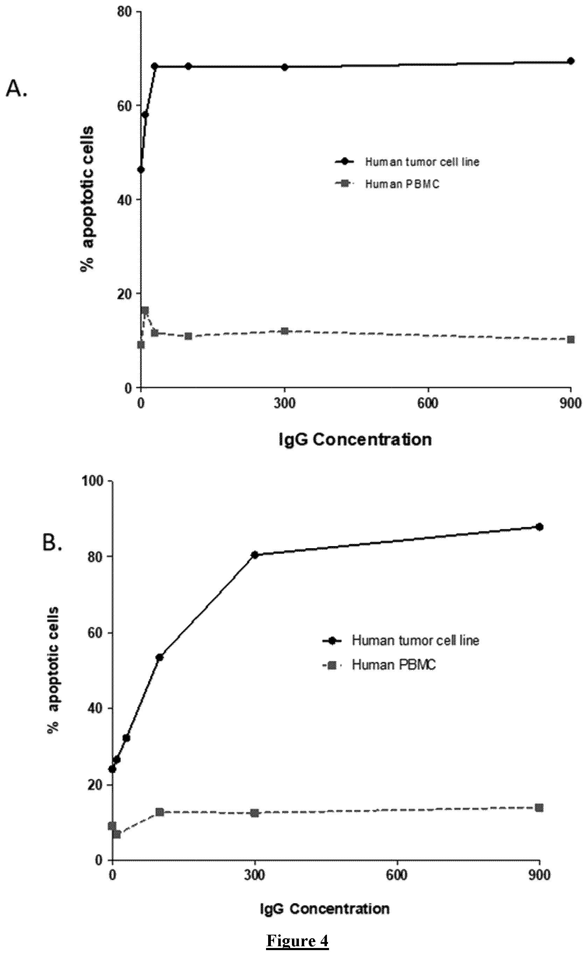

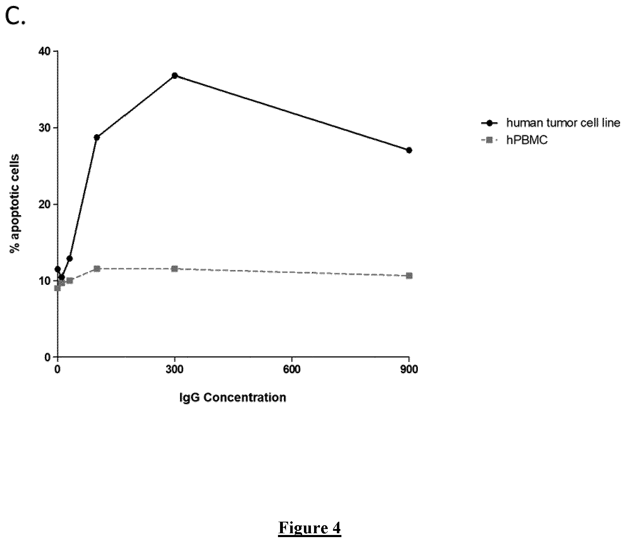

**Figure 4****:** describes the cross reactivity of anti-cancer pAb with normal human PBMC.

The percentage of apoptotic cells was determined by FACS using different concentration of the tested anti-cancer pAb exposed to normal human PBMCs (■).

Human tumor cell line of the corresponding pAb was used as a positive control ( 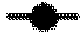 ).

The tested polyclonal antibodies are as follows:
Fig 4A/ XON9.
Fig 4B/ XON5.
Fig 4C/ XON7.

In each Figure 4A to 4C: ordinate: % of apoptotic cells; Abscissa: Concentration of the tested combination of antibodies (pAb) (µg/mL).

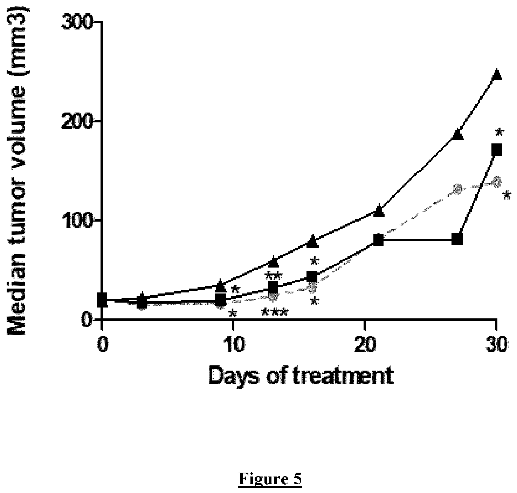

**Figure 5****:** describes *in vivo* efficiency of XON5 or XON7 as defined above on human lung cancer (A549 cell line).

Fig 5 represents the median volume of the human lung tumor in mice model (A549 cell lines) from 0 to 30 days after inoculation and following treatment with XON5 ( 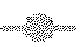 ), XON7 (■) or without treatment w/o (A).

Statistical was performed with ANOVA post-hoc test Newman keuls.

Ordinate: median tumor volume (mm³)

Abscissa: days after inoculation with the tested combination of antibodies (XON5 or XON7).

**Figure 6****:** describes *in vivo* efficiency of XON5 or XON7 as defined above on human breast cancer (MDA-MB-231 cell line).

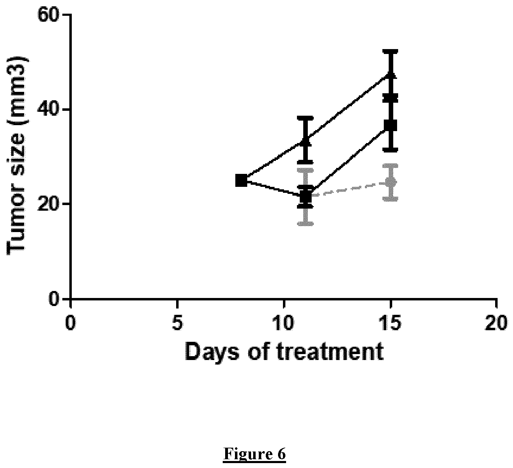

Fig 6 represents the volume of the human breast cancer model (MDA-MB231 cell lines) from 0 to 20 days after inoculation and following treatment with XON5 ( 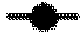 ) or XON7 (■) or with the non-immune IgG (·) as control.

Ordinate: Tumor volume (mm³)

Abscissa: days after inoculation with the tested combination of antibodies.

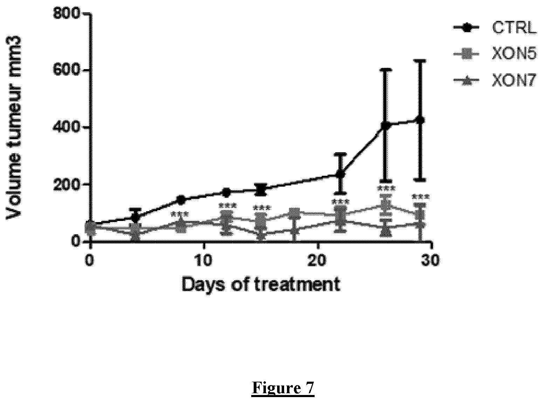

**Figure 7****:** describes *in vivo* efficiency of XON5 or XON7 on human prostate cancer (LNPAC cell line).

Fig 7 represents the median volume of the human prostate tumor in mice model (LNPAC cell line) from 0 to 28 days after inoculation and following treatment with XON5 (■), with XON7 -(A) or without treatment w/o (CTRL - 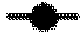 ).

Statistical was performed with one way ANOVA.

*** p<0,001

Ordinate: median tumor volume (mm³).

Abscissa: days after inoculation with the tested polyclonal antibodies.

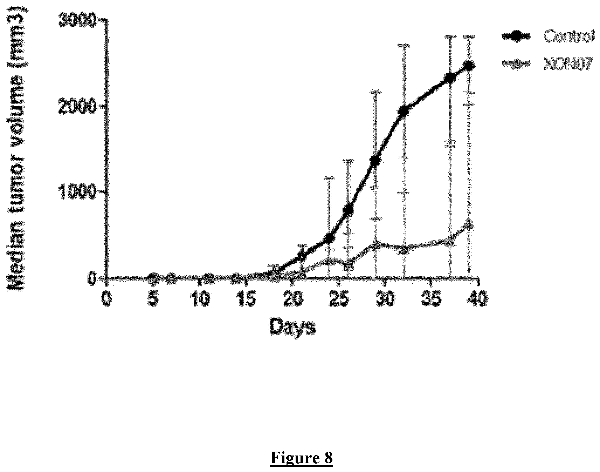

**Figure 8****:** describes *in vivo* efficiency of XON7 as defined above on human colon adenocarcinoma (HCT-116 cell line).

Fig 8 represents the median volume of the human colon adenocarcinoma in mice model (HCT-116 cell line) from 0 to 38 days after inoculation and following treatment with XON7 (·) or without treatment w/o (CTRL - 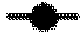 ).

Ordinate: median tumor volume (mm³)

Abscissa: days after inoculation with the tested combination of antibodies.

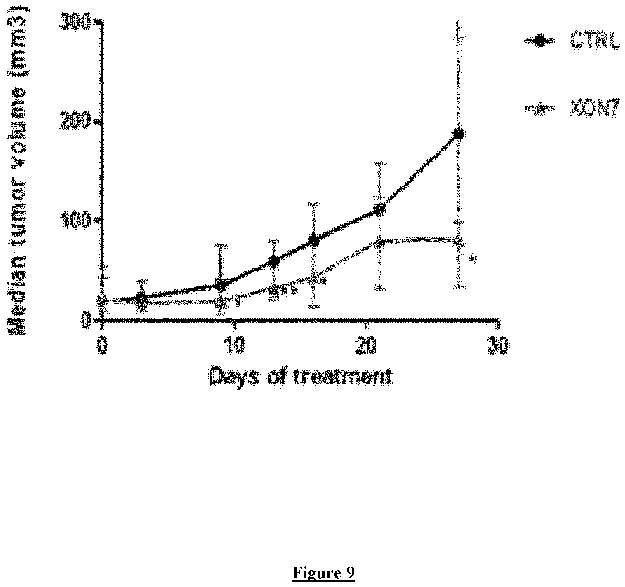

**Figure 9****:** describes *in vivo* efficiency of the XON7 on human T Cell lymphoma (T1301 cell line).

Fig 9 represents the median volume of the human T Cell lymphoma in mice model (T1301 cell line) from 0 to 28 days after inoculation and following treatment with XON7 (▲) or without treatment w/o (CTRL - 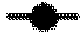 ).

T test - ** p<0,01, * p<0,05

Ordinate: median tumor volume (mm³)

Abscissa: days after inoculation with the tested polyclonal antibodies.

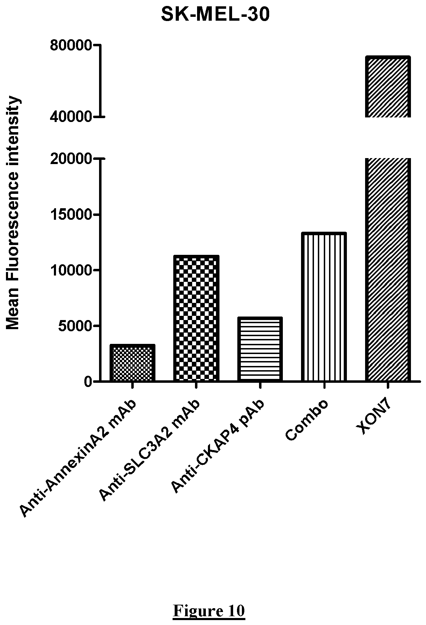

**Figure 10****:** describes an *in vitro* binding assay by NucleoCounter^{®} NC-3000^{™} Advanced Image Cytometer (Chemometec, Denmark) of various monoclonal or polyclonal antibodies and combinations of antibodies on a human melanoma cell line (SK-MEL-30). An Alexa Fluor 488 protein A was used as secondary antibody.

The various implemented antibodies or combinations of antibodies are (from left to right on the abscissa axis):
- an anti-Annexin A2 monoclonal antibody;
- an anti-SLC3A2 monoclonal antibody;
- an anti-CKAP4 polyclonal antibody (i.e. a group of antibodies targeting different epitopes of CKAP4);
- a combination of the anti-Annexin A2 monoclonal antibody, the anti-SLC3A2 monoclonal antibody and the anti-CKAP4 polyclonal antibody; and
- XON7.

Ordinate: Mean Fluorescence intensity.

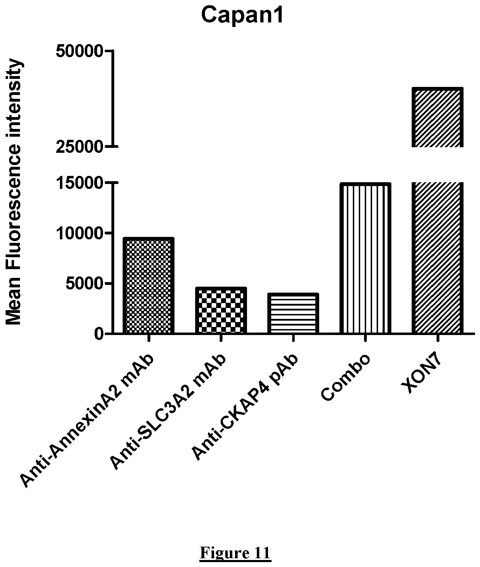

**Figure 11****:** describes an *in vitro* binding assay by NucleoCounter^{®} NC-3000^{™} Advanced Image Cytometer (Chemometec, Denmark) of various monoclonal or polyclonal antibodies and combinations of antibodies on a human pancreatic adenocarcinoma cell line (CAPAN-1). An Alexa Fluor 488 protein A was used as secondary antibody.

The various implemented antibodies or combinations of antibodies are (from left to right on the abscissa axis):
- an anti-Annexin A2 monoclonal antibody;
- an anti-SLC3A2 monoclonal antibody;
- an anti-CKAP4 polyclonal antibody (i.e. a group of antibodies targeting different epitopes of CKAP4);
- a combination of the anti-Annexin A2 monoclonal antibody, the anti-SLC3A2 monoclonal antibody and the anti-CKAP4 polyclonal antibody; and
- XON7.

Ordinate: Mean Fluorescence intensity.

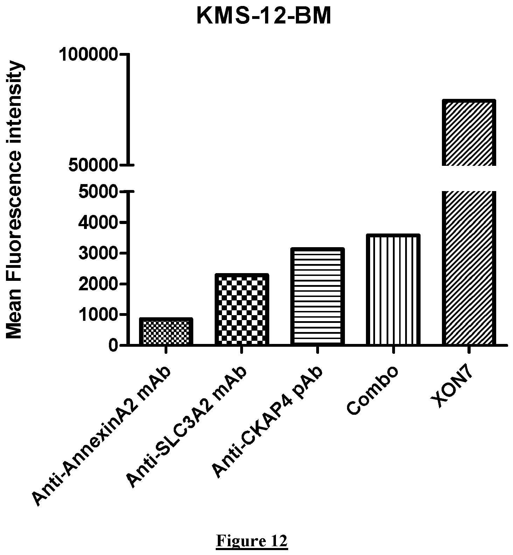

**Figure 12****:** describes an *in vitro* binding assay by NucleoCounter^{®} NC-3000^{™} Advanced Image Cytometer (Chemometec, Denmark) of various monoclonal or polyclonal antibodies and combinations of antibodies on a human myeloma cell line (KMS-12-BM). An Alexa Fluor 488 protein A was used as secondary antibody.

The various implemented antibodies or combinations of antibodies are (from left to right on the abscissa axis):
- an anti-Annexin A2 monoclonal antibody;
- an anti-SLC3A2 monoclonal antibody;
- an anti-CKAP4 polyclonal antibody (i.e. a group of antibodies targeting different epitopes of Annexin A2);
- a combination of the anti-Annexin A2 monoclonal antibody, the anti-SLC3A2 monoclonal antibody and the anti-CKAP4 polyclonal antibody; and
- XON7.

Ordinate: Mean Fluorescence intensity.

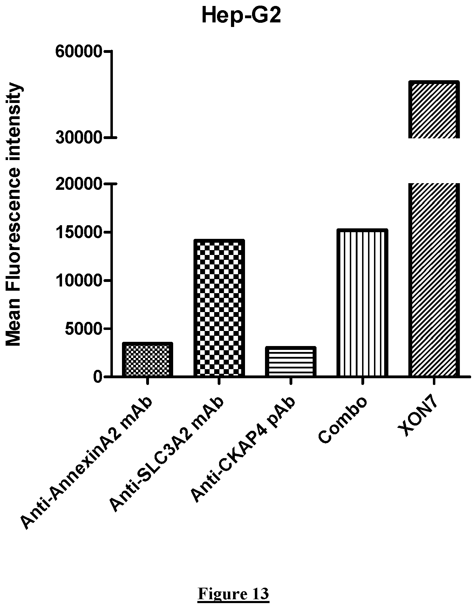

**Figure 13****:** describes an *in vitro* binding assay by NucleoCounter^{®} NC-3000^{™} Advanced Image Cytometer (Chemometec, Denmark) of various monoclonal or polyclonal antibodies and combinations of antibodies on a human hepatocarcinoma cell line (HEP-G2). An Alexa Fluor 488 protein A was used as secondary antibody.

The various implemented antibodies or combinations of antibodies are (from left to right on the abscissa axis):
- an anti-Annexin A2 monoclonal antibody;
- an anti-SLC3A2 monoclonal antibody;
- an anti-CKAP4 polyclonal antibody (i.e. a group of antibodies targeting different epitopes of CKAP4);
- a combination of the anti-Annexin A2 monoclonal antibody, the anti-SLC3A2 monoclonal antibody and the anti-CKAP4 polyclonal antibody; and
- XON7.

Ordinate: Mean Fluorescence intensity.

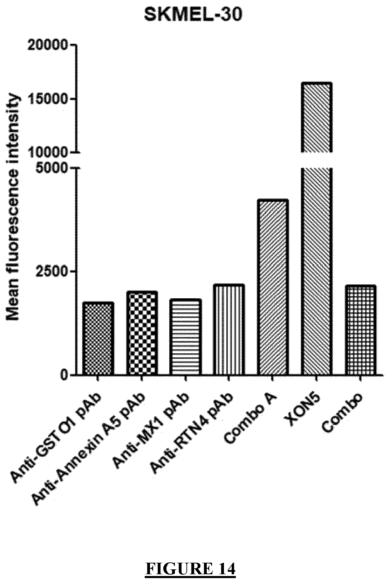

**Figure 14****:** describes an *in vitro* binding assay by NucleoCounter^{®} NC-3000^{™} Advanced Image Cytometer (Chemometec, Denmark) of various monoclonal or polyclonal antibodies and combinations of antibodies on a human myeloma cell line (KMS-12-BM). An Alexa Fluor 488 protein A was used as secondary antibody.

The various implemented antibodies or combinations of antibodies are (from left to right on the abscissa axis):
- an anti-GSTO1 polyclonal antibody (i.e. a group of antibodies targeting different epitopes of GSTO1);
- an anti-Annexin A5 polyclonal antibody (i.e. a group of antibodies targeting different epitopes of Annexin A5);
- an anti-MX1 polyclonal antibody (i.e. a group of antibodies targeting different epitopes of MX1);
- an anti-RNT4 polyclonal antibody (i.e. a group of antibodies targeting different epitopes of RNT4);
- a combination ("Combo A") of the anti-GSTO1 polyclonal antibody, the anti-Annexin A5 polyclonal antibody, the anti-MX1 polyclonal antibody and the anti-RTN4 polyclonal antibody;
- a combination ("combo") of the anti-Annexin A2 monoclonal antibody, the anti-SLC3A2 monoclonal antibody and the anti-CKAP4 polyclonal antibody;
   and
- XON5.

Ordinate: Mean Fluorescence intensity.

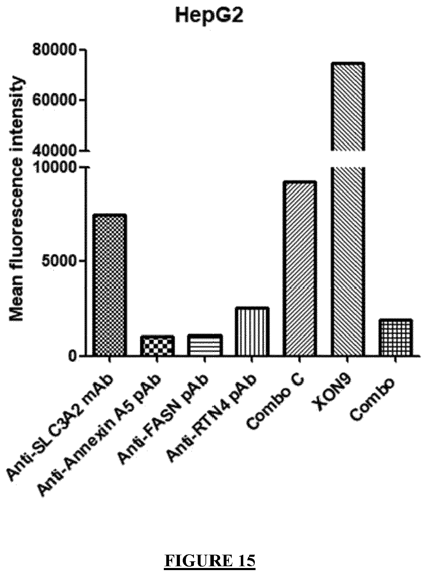

**Figure 15****:** describes an *in vitro* binding assay by NucleoCounter^{®} NC-3000^{™} Advanced Image Cytometer (Chemometec, Denmark) of various monoclonal or polyclonal antibodies and combinations of antibodies on a human hepatocarcinoma cell line (HEP-G2). An Alexa Fluor 488 protein A was used as secondary antibody.

The various implemented antibodies or combinations of antibodies are (from left to right on the abscissa axis):
- an anti-SLC3A2 monoclonal antibody;
- an anti-Annexin A5 polyclonal antibody(i.e. a group of antibodies targeting different epitopes of Annexin A5);
- an anti-FASN polyclonal antibody (i.e. a group of antibodies targeting different epitopes of FASN);
- an anti-RNT4 polyclonal antibody (i.e. a group of antibodies targeting different epitopes of RNT4);
- a combination ("Combo C") of the anti- SLC3A2 monoclonal antibody, the anti-Annexin A5 polyclonal antibody, the anti-FASN polyclonal antibody and the anti-RTN4 polyclonal antibody;
- a combination ("Combo") of the anti-Annexin A2 monoclonal antibody, the anti-SLC3A2 monoclonal antibody and the anti-CKAP4 polyclonal antibody;
   and
- XON9.

Ordinate: Mean Fluorescence intensity.

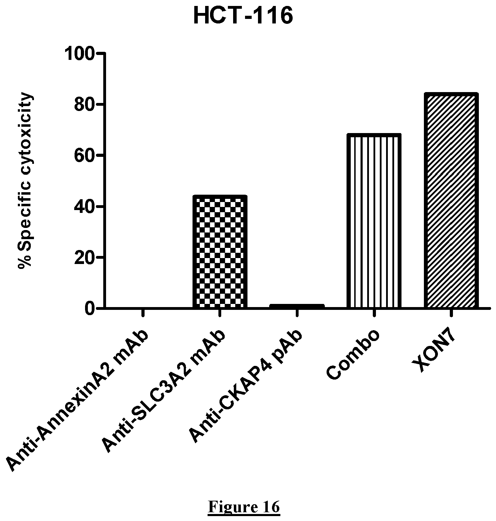

**Figure 16****:** describes *in vitro* complement dependent cytotoxicity (CDC) activity of various monoclonal or polyclonal antibodies and combinations of antibodies on a human colon adenocarcinoma cell line (HCT-116).

The various implemented antibodies or combinations of antibodies are (from left to right on the abscissa axis):
- an anti-Annnexin A2 monoclonal antibody;
- an anti-SLC3A2 monoclonal antibody;
- an anti-CKAP4 polyclonal antibody (i.e. a group of antibodies targeting different epitopes of CKAP4);
- a combination of the anti-Annexin A2 monoclonal antibody, the anti-SLC3A2 monoclonal antibody and the anti-CKAP4 polyclonal antibody; and
- XON7 in presence of rabbit complement (dilution 1/3 final).

Ordinate: % specific cytotoxicity.

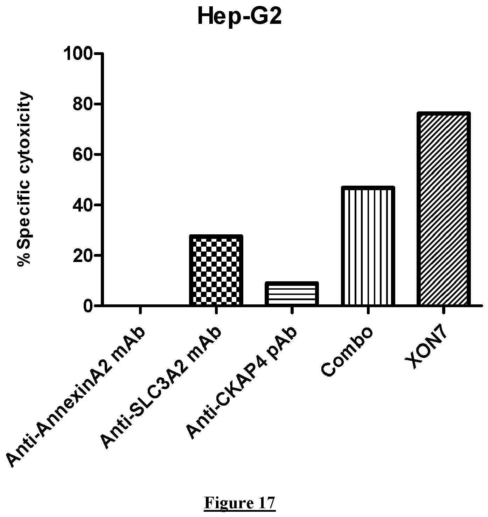

**Figure 17****:** describes *in vitro* complement dependent cytotoxicity (CDC) activity of various monoclonal or polyclonal antibodies and combinations of antibodies on a human hepatocarcinoma cell line (HEP-G2).

The various implemented antibodies or combinations of antibodies are (from left to right on the abscissa axis):
- an anti-Annexin A2 monoclonal antibody;
- an anti-SLC3A2 monoclonal antibody;
- an anti-CKAP4 polyclonal antibody (i.e. a group of antibodies targeting different epitopes of CKAP4);
- a combination of the anti-Annexin A2 monoclonal antibody, the anti-SLC3A2 monoclonal antibody and the anti-CKAP4 polyclonal antibody; and
- XON7 in presence of rabbit complement (dilution 1/3 final).

Ordinate: % specific cytotoxicity.

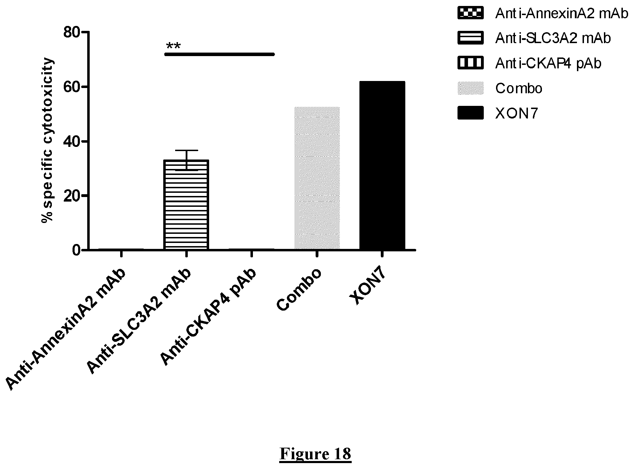

**Figure 18****:** describes *in vitro* complement dependent cytotoxicity (CDC) activity of various monoclonal or polyclonal antibodies and combinations of antibodies on a human myeloma cell line (KMS-12-BM).

The various implemented antibodies or combinations of antibodies are (from left to right on the abscissa axis):
- an anti-Annexin A2 monoclonal antibody;
- an anti-SLC3A2 monoclonal antibody;
- an anti-CKAP4 polyclonal antibody (i.e. a group of antibodies targeting different epitopes of CKAP4);
- a combination of the anti-Annexin A2 monoclonal antibody, the anti-SLC3A2 monoclonal antibody and the anti-CKAP4 polyclonal antibody; and
- XON7 in presence of rabbit complement (dilution 1/3 final).
- negative control

Ordinate: % specific cytotoxicity.

One way-Anova - Post-Hoc Newman Keuls **p<0,05

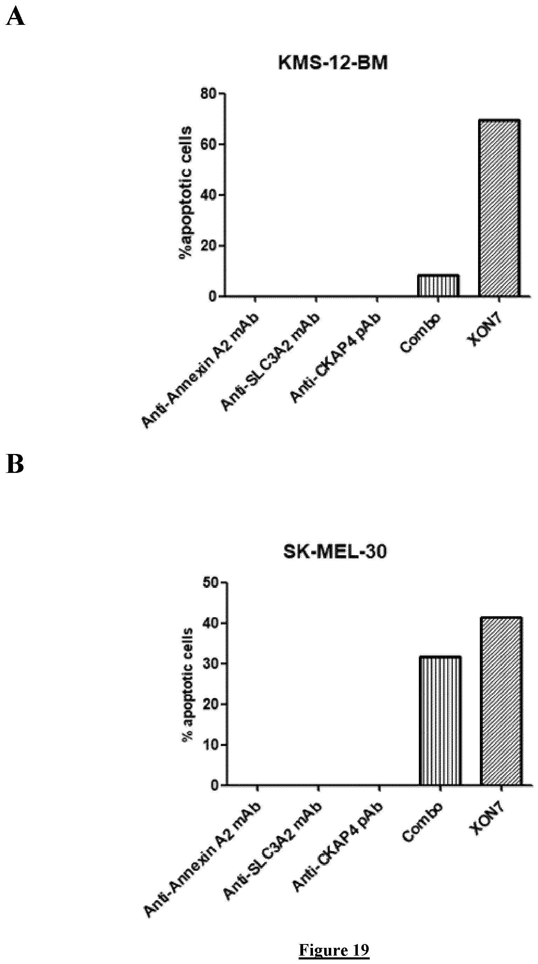

**Figure 19****:** describes *in vitro* apoptotic activity of various monoclonal or polyclonal antibodies and combinations of antibodies on a human myeloma cell line (KMS-12-BM) (Figure 19A) or a human melanoma cell line (SK-MEL-30) (Figure 19B).

The various implemented antibodies or combinations of antibodies are (from left to right on the abscissa axis):
- an anti-Annexin A2 monoclonal antibody;
- an anti-SLC3A2 monoclonal antibody;
- an anti-CKAP4 polyclonal antibody (i.e. a group of antibodies targeting different epitopes of CKAP4);
- a combination of the anti-Annexin A2 monoclonal antibody, the anti-SLC3A2monoclonal antibody and the anti-CKAP4 polyclonal antibody; and
- XON7 in presence of rabbit complement (dilution 1/3 final).
- negative control

Ordinate: % apoptotic cells.

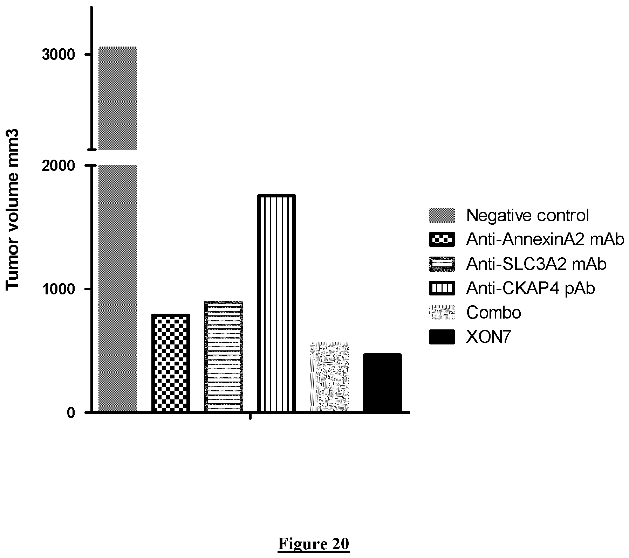

**Figure 20****:** represents the median volume of a multiple myeloma in mice model (KMS-12-BM) from 0 to 28 days after inoculation (intraperitoneal injection) and following treatment with various monoclonal or polyclonal antibodies and combinations of antibodies on a human myeloma cell line (KMS-12-BM).

The various implemented antibodies or combinations of antibodies are (from left to right on the abscissa axis):
- negative control: no treatment;
- an anti-Annexin A2 monoclonal antibody;
- an anti-SLC3A2 monoclonal antibody
- an anti-CKAP4 polyclonal antibody (i.e. a group of antibodies targeting different epitopes of CKAP4);
- a combination of the anti-Annexin A2 , the anti-SLC3A2 and the anti-CKAP4 antibodies; and
- XON7.

Ordinate: tumor volume mm³.

### Detailed description of the invention

### 1. Definitions

Several definitions are set forth below. Such definitions are meant to encompass grammatical equivalents.

Unless otherwise defined herein, scientific and technical terms used in connection with the present invention shall have the meanings that are commonly understood by those of ordinary skill in the art. For example, the Concise Dictionary of Biomedicine and Molecular Biology, Juo, Pei-Show, 2nd ed., 2002, CRC Press; The Dictionary of Cell and Molecular Biology, 3rd ed., 1999, Academic Press; and the Oxford Dictionary Of Biochemistry And Molecular Biology, Revised, 2000, Oxford University Press, may provide one of skill with a general dictionary of many of the terms used in this disclosure. In case of conflict, the present specification, including definitions, will control. Exemplary methods and materials are described below, although methods and materials similar or equivalent to those described herein can also be used in the practice or testing of the present invention. Units, prefixes, and symbols are denoted in their Système International des Unités (SI) accepted form. The headings provided herein are not limitations of the various aspects of the disclosure.

All publications and other references mentioned herein are incorporated by reference in their entirety.

It is to be noted that the term *"a"* or *"an"* entity refers to one or more of that entity; for example, *"an antibody,"* is understood to represent one or more antibodies. As such, the terms "*a*" (or *"an*"), *"one or more,"* and *"at least one"* can be used interchangeably herein.

Throughout this specification and embodiments, the words *"have"* and *"comprise,"* or variations such as *"has*," *"having," "comprises,"* or *"comprising,"* will be understood to imply the inclusion of a stated integer or group of integers but not the exclusion of any other integer or group of integers. The words *"have"* and *"comprise,"* or variations such as *"has," "having," "comprises,"* or *"comprising,"* will be understood to imply the inclusion of the stated element(s) (such as a composition of matter or a method step) but not the exclusion of any other elements. The term *"consisting of'* implies the inclusion of the stated element(s), to the exclusion of any additional elements. The term *"consisting essentially of*" implies the inclusion of the stated elements, and possibly other element(s) where the other element(s) do not materially affect the basic characteristic(s) of the disclosure. It is understood that the different embodiments of the disclosure using the term *"comprising"* or equivalent cover the embodiments where this term is replaced with *"comprising only", "consisting of* or *"consisting essentially of'.*

It is understood that wherever aspects are described herein with the language *"comprising,"* otherwise analogous aspects described in terms of *"consisting of*" and/or *"consisting essentially of*" are also provided.

Furthermore, *"and*/*or"* where used herein is to be taken as specific disclosure of each of the two specified features or components with or without the other. Thus, the term "and/or" as used in a phrase such as "A and/or B" herein is intended to include "A and B," "A or B," "A" (alone), and "B" (alone). Likewise, the term "and/or" as used in a phrase such as "A, B, and/or C" is intended to encompass each of the following aspects: A, B, and C; A, B, or C; A or C; A or B; B or C; A and C; A and B; B and C; A (alone); B (alone); and C (alone).

*"Pharmaceutically"* or *"pharmaceutically acceptable"* refers to molecular entities and compositions that do not produce an adverse, allergic or other untoward reaction when administered to a mammal, especially a human, as appropriate. A pharmaceutically acceptable carrier or excipient refers to a non-toxic solid, semi-solid or liquid filler, diluent, encapsulating material or formulation auxiliary of any type.

As used herein, *"pharmaceutically-acceptable carriers"* includes any and all solvents, dispersion media, coatings, antibacterial and antifungal agents, and the like that are physiologically compatible. Examples of suitable carriers, diluents and/or excipients include one or more of water, amino acids, saline, phosphate buffered saline, buffer phosphate, acetate, citrate, succinate; amino acids and derivates such as histidine, arginine, glycine, proline, glycylglycine; inorganic salts NaCl, calcium chloride; sugars or polyalcohols such as dextrose, glycerol, ethanol, sucrose, trehalose, mannitol; surfactants such as Polysorbate 80, polysorbate 20, poloxamer 188; and the like, as well as combination thereof. In many cases, it will be preferable to include isotonic agents, such as sugars, polyalcohols, or sodium chloride in the composition, and formulation may also contain an antioxidant such as tryptamine and a stabilizing agent such as Tween 20. Suitable excipients, as well as pharmaceutical formulation requirements, are described in "Remington: The Science & Practice of Pharmacy", which is a reference work in the field.

As used herein the term "antibody" have the same meaning and will be used equally in the present description. The term "antibody" as used herein refers to isolated or recombinant immunoglobulin molecules and immunologically active portions of immunoglobulin molecules, i.e. molecules that contain an antigen binding site that immunospecifically binds an antigen, such as proteins SLC3A2, CKAP4, Annexin A2, GSTO1, Annexin A5, MX1, RTN4, FASN or PDIA4. As such, the term antibody encompasses not only whole antibody molecules, but also antibody fragments as well as variants (including derivatives) of antibodies and antibody fragments. In natural antibodies, two heavy chains are linked to each other by disulphide bonds and each heavy chain is linked to a light chain by a disulphide bond. There are two types of light chain, lambda (1) and kappa (k). There are five main heavy chain classes (or isotypes) which determine the functional activity of an antibody molecule: IgM, IgD, IgG, IgA and IgE. Each chain contains distinct sequence domains. The light chain includes two domains, a variable domain (VL) and a constant domain (CL). The heavy chain includes four domains, a variable domain (VH) and three constant domains (CH1, CH2 and CH3, collectively referred to as CH). The variable regions of both light (VL) and heavy (VH) chains determine binding recognition and specificity to the antigen. The constant region domains of the light (CL) and heavy (CH) chains confer important biological properties such as antibody chain association, secretion, trans-placental mobility, complement binding, and binding to Fc receptors (FcR). The Fv fragment is the N-terminal part of the Fab fragment of an immunoglobulin and consists of the variable portions of one light chain and one heavy chain. The specificity of the antibody resides in the structural complementarity between the antibody combining site and the antigenic determinant. Antibody combining sites are made up of residues that are primarily from the hypervariable or complementarity determining regions (CDRs). Occasionally, residues from nonhypervariable or framework regions (FR) can participate to the antibody binding site or influence the overall domain structure and hence the combining site. Complementarity Determining Regions or CDRs refer to amino acid sequences which together define the binding affinity and specificity of the natural Fv region of a native immunoglobulin binding site. The light and heavy chains of an immunoglobulin each have three CDRs, designated CDR1-L, CDR2-L, L- CDR3-L and CDR1-H CDR2-H, CDR3-H, respectively. An antigen-binding site, therefore, typically includes six CDRs, comprising the CDR set from each of a heavy and a light chain V region. Framework Regions (FRs) refer to amino acid sequences interposed between CDRs.

The term "antibody" includes, but is not limited to, monoclonal antibodies, polyclonal antibodies, human antibodies, humanized antibodies, camelid antibodies and chimeric antibodies. The antibodies can be of any isotype/class (e.g., IgG, IgE, IgM, IgD, IgA and IgY), or subclass (e.g., IgGl, IgG2, IgG3, IgG4, IgAl and IgA2).

In the context of the present disclosure, the term *"antibody"* specifically includes an antibody binding a SLC3A2 protein (also termed an anti-SLC3A2 antibody) and/or an antibody binding a CKAP4 protein (also termed an anti-CKAP4 antibody) and/or an antibody binding a Annexin A2 protein (also termed an anti-Annexin A2 antibody) and/or an antibody binding a GSTO1 protein (also termed an anti-GSTO1 antibody) and/or an antibody binding a Annexin A5 protein (also termed an anti-Annexin A5 antibody) and/or an antibody binding a MX1 protein (also termed an anti-MX1 antibody) and/or an antibody binding a RTN4 protein (also termed an anti- RTN4 antibody) and/or an antibody binding a FASN protein (also termed an anti-FASN antibody) and/or an antibody binding a PDIA4 protein (also termed an anti-PDIA4 antibody).

It is intended that all references to the term *"antibody"* or *"antigen-binding fragment thereof* as used herein, referred to the antibodies or the antigen-binding fragments thereof as described in the present disclosure.

*"Fragments"* of (conventional) antibodies comprise a portion of an intact antibody, in particular the antigen binding region or variable region of the intact antibody. Examples of antibody fragments include Fv, Fab, F(ab')2, Fab', dsFv, (dsFv)2, scFv, sc(Fv)2, diabodies, bispecific and multispecific antibodies formed from antibody fragments. A fragment of a conventional antibody may also be a single domain antibody, such as a heavy chain antibody or VHH.

The term "Fab" denotes an antibody fragment having a molecular weight of about 50,000 and antigen binding activity, in which about a half of the N-terminal side of the heavy chain and the entire light chain are bound together through a disulfide bond. It is usually obtained among fragments by treating IgG with a protease, papain.

The term "F(ab')2" refers to an antibody fragment having a molecular weight of about 100,000 and antigen binding activity, which is slightly larger than 2 identical Fab fragments bound via a disulfide bond of the hinge region. It is usually obtained among fragments by treating IgG with a protease, pepsin.

The term "Fab'" refers to an antibody fragment having a molecular weight of about 50,000 and antigen binding activity, which is obtained by cutting a disulfide bond of the hinge region of the F(ab')2.

A single chain Fv ("scFv") polypeptide is a covalently linked VH::VL heterodimer which is usually expressed from a gene fusion including VH and VL encoding genes linked by a peptide-encoding linker. The human scFv fragment of the disclosure includes CDRs that are held in appropriate conformation, in particular by using gene recombination techniques. Divalent and multivalent antibody fragments can form either spontaneously by association of monovalent scFvs, or can be generated by coupling monovalent scFvs by a peptide linker, such as divalent sc(Fv)2. "dsFv" is a VH::VL heterodimer stabilized by a disulphide bond. "(dsFv)2" denotes two dsFv coupled by a peptide linker.

The term "bispecific antibody" or "BsAb" denotes an antibody which combines the antigen-binding sites of two antibodies within a single molecule. Thus, BsAbs are able to bind two different antigens simultaneously. Genetic engineering has been used with increasing frequency to design, modify, and produce antibodies or antibody derivatives with a desired set of binding properties and effector functions as described for instance in EP 2 050 764 A1.

The term *"multispecific antibody"* denotes an antibody which combines the antigen-binding sites of two or more antibodies within a single molecule.

The term "diabodies" refers to small antibody fragments with two antigen-binding sites, which fragments comprise a heavy-chain variable domain (VH) connected to a light-chain variable domain (VL) in the same polypeptide chain (VH-VL). By using a linker that is too short to allow pairing between the two domains of the same chain, the domains are forced to pair with the complementary domains of another chain and create two antigen-binding sites.

Antibodies of compositions according to the invention may be produced by any technique known in the art, such as, without limitation, any chemical, biological, genetic or enzymatic technique, either alone or in combination. The antibodies of the present invention may comprise polyclonal antibodies. Methods of preparing polyclonal antibodies are known to the skilled artisan (Harlow et al., Antibodies: a Laboratory Manual, Cold Spring Harbor Laboratory Press, 2nd ed. (1988)).

The term "monoclonal antibody" or "mAb" as used herein refers to an antibody molecule of a single amino acid sequence, which is directed against a specific antigen, and is not to be construed as requiring production of the antibody by any particular method. A monoclonal antibody may be produced by a single clone of B cells or hybridoma, but may also be recombinant, i.e., produced by protein engineering.

By *"polyclonal antibodies"* or *"polyclonal antibody"* as used herein is meant a mixture of antibodies recognizing different epitopes of a given antigen, or even different epitopes of different antigens expresses by a given cell or group of cells. Polyclonal antibodies encompass those which are contained in, or alternatively which are derived from, body fluids, especially serum or plasma from a non-human mammal organism, in particular from a pig.

The term "humanized antibody" refers to an antibody which is wholly or partially of non-human origin, and which has been modified to replace certain amino acids, in particular in the framework regions of the VH and VL domains, in order to avoid or minimize an immune response in humans. The constant domains of a humanized antibody are most of the time human CH and CL domains.

The term "recombinant" as applied to an antibody, or an antigen-binding fragment thereof, a nucleic acid sequence, an expression vector or a host cell means that those are the products of various combinations *of in vitro* cloning, restriction, ligation steps, and other genetic engineering procedures.

In the context of the present invention, the term *"antibody"* in particular encompasses an antibody from a pig, and more particularly an antibody from pig, the said antibody being devoid of at least one antigenic determinant selected from (i) N-glycolylneuraminic acid (Neu5Gc) and (ii) α-1,3-galactose, and in particular is devoid of the two antigenic determinants N-glycolylneuraminic acid (Neu5Gc) and α-1,3-galactose; and which further preferably comprises at least one sugar moiety distinct from the antigenic determinants (i) N-glycolylneuraminic acid (Neu5Gc) and/or (ii) α-1,3-galactose.

The term "antigen" as used in the present disclosure refers to a molecule or a portion of a molecule capable of being bound by one or more antibodies. An antigen can have one or more than one epitope. For instance, in the context of the disclosure, an antigen is in particular selected from the group consisting of proteins SLC3A2, CKAP4, Annexin A2, GSTO1, Annexin A5, MX1, RTN4, FASN and PDIA4.

The term *"affinity",* as used herein, means the strength of the binding of an antibody to an epitope presented on an antigen. The affinity of an antibody is given by the dissociation constant KD, defined as [Ab] x [Ag] / [Ab-Ag], where [Ab-Ag] is the molar concentration of the antibody-antigen complex, [Ab] is the molar concentration of the unbound antibody and [Ag] is the molar concentration of the unbound antigen. The affinity constant Ka is defined by 1/Kd. Methods for determining the affinity of Abs can be found in, for example, Harlow, et al., Cold Spring Harbor Laboratory Press, Cold Spring Harbor, N.Y., 1988, Coligan et al., Current Protocols in Immunology, Greene Publishing Assoc. and Wiley Interscience, N.Y., (1992, 1993), or Müller, Methods Enzymol. 1983;92:589-601, which references are entirely incorporated herein by reference. A preferred and standard method well known in the art for determining the affinity of mAbs is the measurement of surface plasmon resonance) by using the Biacore instruments (Laure et al. Curr Protoc Protein Sci. 2006 Sep;Chapter 19:Unit 19.13). For multimeric antigens, such as virus capsids, to which both antigen binding sites of an antibody can bind simultaneously, Ka and KD values determined by such standard methods measure the functional affinity, or avidity of the interaction. Illustratively, an antibody is considered to bind an antigen when a functional binding affinity (KD), preferably measured by surface plasmon resonance, is of 10-8 mol/l (M) or less, preferably 10-9 M to 10-12 M.

By *"IgG"* as used herein is meant a polypeptide belonging to the class of antibodies that are substantially encoded by a recognized immunoglobulin gamma gene. In humans, IgG comprises the subclasses or isotypes IgG1, IgG2, IgG3, and IgG4. In mice, IgG comprises IgG1, IgG2a, IgG2b, IgG3. In pigs, immunoglobulins comprise the classes or isotypes IgM, IgD, IgG, IgE and IgA antibodies and the IgG isotype comprise 11 subclasses (Butler et al., Developmental and Comparative Immunology 30 (2006) 199-221; Butler et al., Developmental and Comparative Immunology 33 (2009) 321-333). Full-length IgGs consist of two identical pairs of two immunoglobulin chains, each pair having one light and one heavy chain, each light chain comprising immunoglobulin domains VL and CL, and each heavy chain comprising immunoglobulin domains VH, Cγ1 (also called CH1), Cγ2 (also called CH2), and Cγ3 (also called CH3).

By *"antigenic determinant"* (or epitope), as applied herein to pig antibodies, as used herein is meant a structural component of an antigenic molecule, which includes an antigenic protein and an antigenic carbohydrate, responsible for its specific interaction with antibody molecules elicited by the same or related antigen. By extension, the term "antigenic determinant", as applied herein to pig antibodies is also used collectively herein for an antigenic molecule comprising a plurality of epitopes, including conformational motives in which the sugar moiety is needed but represent only part of the epitope, susceptible to be recognized by antibody molecules elicited by the same or related antigen. Illustratively, the antigenic molecule N-glycolylneuraminic acid (Neu5Gc) may be called herein an "antigenic determinant", although the said antigenic molecule may exhibit more than one epitope recognized by antibodies elicited with Neu5Gc or with Neu5Gc containing molecules.

By *"conventional polyclonal antibodies",* as used herein is meant polyclonal antibodies, and in particular pig or rabbit polyclonal antibodies, that are not devoid of the antigenic determinants N-glycolylneuraminic acid (Neu5Gc) and α-1,3-galactose. In this regard, it may be notably cited the products commercialized under the name Thymoglobulin, Grafalon, Atgam or p-ALG^{®}.

The term *"cancer"* is herein used in its traditional sense, and refers to a cell or group of cells displaying uncontrolled growth, invasion upon adjacent tissues.

Examples of cancers more particularly considered in the present text are provided further. Cancers may in particular refer herein to a cancer selected from the group consisting of myeloma; melanoma; breast cancer; prostate cancer; colorectal cancer, in particular a colon cancer; lung cancer, in particular non small cell lung cancer; liver cancer, in particular an hepatocarcinoma; pancreas cancer; lymphoma, in particular B-cell or T-cell lymphoma, more particularly T-cell lymphoma; gastric cancer; head and neck cancer; ovarian cancer; sarcoma; leukemia, osteosarcoma; anal cancer; testicular cancer; uterus cancer; thyroid cancer; cancer of the central nervous system; gastrointestinal stromal cancer; epidermal carcinoma and oesophageal cancer.

By a *"therapeutically effective amount"* of a combination of antibodies used according to the invention is meant a sufficient amount of the antibodies to obtain the desired effect as presented in the present text, and in particular for treating a human subject affected with a cancer. It will be understood, however, that the total daily usage of the combination of antibodies implemented according to the invention will be decided by the attending physician within the scope of sound medical judgment. The specific therapeutically effective dose level for any particular patient will depend upon a variety of factors including the nature of the cancer being treated; the activity of the specific antibodies employed; the specific composition employed; the age, body weight, general health, sex and diet of the patient; the time of administration, route of administration, and rate of excretion of the specific antibodies employed; the duration of the treatment; drugs used in combination or coincidental with the specific antibodies employed; and like factors well known in the medical arts. For example, it is well known within the skill of the art to start doses of the compound at levels lower than those required to achieve the desired therapeutic effect and to gradually increase the dosage until the desired effect is achieved.

The terms *"treatment", "treating"* or *"therapy"* refers to administering an active agent with the purpose to cure, heal, alleviate, relieve, alter, remedy, ameliorate, improve, or affect a condition (i.e., a cancer), the symptoms of the cancer, or to prevent or delay the onset of the symptoms, complications, biochemical indicia of a disease, or otherwise arrest or inhibit further development of the cancer.

The terms *"wild type animal"* or *"WT animal"* come herein in opposition with a genetically altered animal. For example, by "wild type pig" is meant a pig which is not lacking at least one gene selected in a group comprising (i) a gene encoding a functional cytidine-5'-monophosphate N-acetyl neuraminic acid hydrolase (CMAH) and (ii) a gene encoding a functional α-(1,3)-galactosyltransferase.

As an appropriate method for obtaining polyclonal antibodies which may be present in antibody combinations of implemented according to the present invention, may notably be cited the method of fractionated precipitation with ethanol, with ammonium sulfate, with rivanol, with polyethylene glycol or with caprylic acid, the method by passage through ion exchange columns; other methods can involve affinity columns on protein A or G. The antibodies obtained can be then subjected to conventional treatments for their intravenous administration, for example by enzymatic cleavage treatments plasmin, papain or pepsin. In this regard, may be more particularly cited the protocol implemented in example 3 of EP 0 335 804, which implements an ion exchange chromatography on DEAE cellulose.

Such antibodies can for example be generated through immunization of a non-human animal, in particular a non-human mammal, according to methods well known to the man skilled in the art. The non-human mammal may be selected from the group consisting of rodents, such as mice, rats, guinea pigs and hamsters; lagomorphs, such as rabbits; ferrets; felines, such as cats; canines, such as dogs; goats; sheep; bovines, such as cows; swines, such as pigs and hogs; camelids; horses; and non-human primates. The non-human mammal may more particularly be a pig. Accordingly, by *"anti-cancer pAb from pig origin"* for example, it is meant that the polyclonal antibodies have been obtained through immunization of a pig with a combination of proteins of interest or with a cell naturally expressing, or genetically altered in order to express on its surface, the proteins of interest against which it is desired to obtained polyclonal antibodies. See Reynard et al. PLoS One. 2016; 11(6): e0156775; Schieferdecker et al., Oncotarget. 2016 Oct 11; 7(41): 67061-67070 and Zhang et al. 2014 (DOI: 10.1038/srep04984).

The terms **"polypeptide"** and **"protein"** are used interchangeably herein to refer to a sequence of amino acid residues. The terms apply to amino acid sequences in which one or more amino acid residue is an artificial chemical mimetic of a corresponding naturally occurring amino acid, as well as to naturally occurring amino acid sequences and nonnaturally occurring amino acid sequence.

### 2. Antibody combinations implemented according to the invention

As mentioned above, the present invention relates to the implementation of a combination of antibodies, or antigen-binding fragments thereof, for use in the treatment of a cancer in a human subject in need thereof.

Such combination comprises:
- at least a first antibody, or an antigen-binding fragment thereof,
- at least a second antibody, or an antigen-binding fragment thereof, and
- at least a third antibody, or an antigen-binding fragment thereof,
each of these antibodies independently and specifically binding to an antigen selected from the group consisting of SLC3A2, CKAP4, Annexin A2, GSTO1, Annexin A5, MX1, RTN4, FASN and PDIA4.

The first, the second and the third antibodies, or antigen-binding fragments thereof, each bind to a different antigen selected from the group consisting of SLC3A2, CKAP4, Annexin A2, GSTO1, Annexin A5, MX1, RTN4, FASN and PDIA4. Accordingly, it is understood that, in the context of the present invention, at least three antigens selected from the group consisting of SLC3A2, CKAP4, Annexin A2, GSTO1, Annexin A5, MX1, RTN4, FASN and PDIA4 are bound by antibodies of a combination of antibodies implemented according to the invention.

Each one of the antibodies present in a combination for use according to the invention may, independently from the others, be a monoclonal antibody, a polyclonal antibody or an antigen-binding fragment thereof. As defined above, said antigen-binding fragment may independently be selected from the group consisting of Fv, Fab, F(ab')2, Fab', dsFv, (dsFv)2, scFv, sc(Fv)2, or diabodies.

The combination of antibodies for use according to the invention may comprise antibodies targeting more than three different proteins among the group consisting of SLC3A2, CKAP4, Annexin A2, GSTO1, Annexin A5, MX1, RTN4, FASN and PDIA4. The antibodies present in a combination of antibodies for use according to the invention may accordingly target at least 3 proteins, target 4 proteins, target at least 4 proteins, target 5 proteins, target at least 5 proteins, target 6 proteins, target at least 6 proteins, target 7 proteins, target at least 7 proteins, target 8 proteins, target at least 8 proteins, target 9 proteins or target at least 9 proteins from the group consisting of SLC3A2, CKAP4, Annexin A2, GSTO1, Annexin A5, MX1, RTN4, FASN and PDIA4.

In particular, a combination of antibodies for use according to the invention comprises antibodies targeting 3, 4 or 5, more particularly 4 or 5, proteins selected from the group consisting of SLC3A2, CKAP4, Annexin A2, GSTO1, Annexin A5, MX1, RTN4, FASN and PDIA4.

Some or all of the antibodies of an antibody combination for its use according to the invention may be devoid of at least one antigenic determinant selected from (i) N-glycolylneuraminic acid (Neu5Gc) and (ii) α-1,3-galactose. Some or all of the said antibodies for use according to the invention may in particular be devoid of the two antigenic determinants N-glycolylneuraminic acid (Neu5Gc) and α-1,3-galactose.

A method allowing to identify or characterize such antibodies falls within the general knowledge of a man skilled in the art. A method that may be used by the one skilled in the art for identifying or characterizing antibodies according to the invention includes an Enzyme-linked immunosorbent assay (ELISA) wherein, for example, anti-Neu5Gc antibodies and anti-Gal antibodies are used as detection molecules.

The antibodies from the antibody combination implemented according to the invention may be immunoglobulin G antibodies.

Porcine IgG structure and genetics have for example been described in literature (Butler et al. 2009 - DOI: 10.1007/s00251-009-0356-0). Eleven isotypes called IgG1a, IgG1b, IgG2a, IgG2b, IgG3, IgG4a, IgG4b, IgG5a, IgG5b, IgG6a, IgG6b have been proposed based on analysis of the genomic IgH locus sequences. According to the available knowledge on relative abundance of pig IgG subclasses and Fc domains affinity for Protein A (Butler et al. 2009 - DOI: 10.1007/s00251-009-0356-0), during a conventional purification, the relative composition of the IgG isotypes in pig DKO polyAb is estimated to be >80% pig IgG1a/b, of 11% IgG2a/b, of 5.5% IgG3, of 3% IgG4a/b, the remaining fraction being other isotypes (IgG5-6). No detectable IgM or IgA isotype is present in pig DKO IgG polyclonal antibody preparation after purification using Protein A chromatography.

Protein SLC3A2 belongs to the SLC (solute carriers) carrier proteins consisting of genes that form a 65-membered superfamily. They are mainly involved in molecule/drug delivery and are a frequently referenced research resource in drug resistance studies. A subclassification is made into SLC families according to what type of molecule delivery they are responsible for, and these families include: SLC1 family, SLC2 family.... SLC65 family. In addition, every family has sub-members. For example, the SLC3 family has 2 members: Such as, SLC3a1 and SLC3a2. In general, in the operating mechanism, they act together with members of the SLC7 family, so most studies usually analyze both together. On the other hand, SLC3a2 forms a dimer with SLC7a5 for its functionality and is involved in the delivery of amino acids together. CD98 is a transmembrane protein and is found on the cell surface. Therefore, it can be easily targeted with drugs.

The amino acid sequence of SLC3A2 has for reference GenBank: KAI2560544.1.

Antibodies able to specifically target SLC3A2 (anti-SLC3A2 antibodies) are well known in the art. Can for example be mentioned the monoclonal antibodies sc-390154 (commercialized by Santa Cruz Biotechnology), MA5-29573 (commercialized by ThermoFischer Scientific or the polyclonal antibody 15193-1-AP (commercialized by Proteintech Group Inc). The application WO20172114458 also describes several antibodies directed against SLC3A2 and is incorporated by reference.

Protein CKAP4 (cytoskeleton-associated protein 4) is a Dickkopf1 (DKK1) receptor, DKK1 being a secretory protein that antagonizes oncogenic Wnt signaling by binding to the Wnt coreceptor low-density lipoprotein receptor-related protein 6 (LRP6) and may may also regulate its own signaling to promote cancer cell proliferation.

The amino acid sequence of CKAP4 has for reference NCBI Reference Sequence : NP_006816.2.

Antibodies able to specifically target CKAP4 (anti-CKAP4 antibodies) are well known in the art. Can for example be mentioned the polyclonal antibody 16686-1-AP (commercialized by Proteintech Group Inc), the monoclonal antibody MOB-3287z (commercialized by Creative Biolabs) or the monoclonal antibody sc-393544 (commercialized by Santa Cruz Biotechnology). The application WO2019065747 also describes several antibodies directed against CKAP4 and is incorporated by reference.

Protein Annexin A2 is a multifunctional calcium²⁺ (Ca²⁺) and phospholipidbinding protein that is expressed in a wide spectrum of cells.

The amino acid sequence of Annexin A2 has for reference GenBank: AAH23990.1.

Antibodies able to specifically target Annexin A2 (anti-Annexin A2 antibodies) are well known in the art. Can for example be mentioned the monoclonal antibody 671001 (commercialized by BioLegend), the monoclonal antibody bsm-54021R (commercialized by BIOSS Antibodies) or the polyclonal antibody PA527566 (commercialized by Invitrogen). The application WO2018/021972 also describes several antibodies directed against Annexin A2 and is incorporated by reference.

Protein GSTO1 (glutathione-S-transferase Ω-1) belongs to the Glutathione S-transferases (GSTs) multigene family phase-II detoxifying enzymes activated during the resistance to chemotherapeutic agents in several cancers.

The amino acid sequence of GSTO1 has for reference GenBank AAF7336.1.

Antibodies able to specifically target GSTO1 (anti-GSTO1 antibodies) are well known in the art. Can for example be mentioned the polyclonal antibody PA583382 (commercialized by Invitrogen) or the monoclonal antibody ABIN5596941 (commercialized by Antibodies online).

Protein Annexin A5 is a member of the annexin family. This is a multiprotein family of over 160 proteins that share the property of Ca2+-dependent binding to negatively charged phospholipid surfaces. Annexin A5 in particular binds to phosphatidylserine, which is one of the "eat me" signals at the surface of the apoptotic cell.

The amino acid sequence of Annexin A5 has for reference GenBank KAI2535760.

Antibodies able to specifically target Annexin A5 (anti-Annexin A5 antibodies) are well known in the art. Can for example be mentioned the polyclonal antibody 157251131 (commercialized by Fischer Scientific), the polyclonal antibody A304-788A (commercialized by Bethyl Laboratories) or the monoclonal antibody abx100729 (commercialized by Abbexa Ltd).

Protein MX1 (Myxovirus resistance 1) is a GTPase that plays an important role in the defense of mammalian cells against influenza A and other viruses. The Mx1 protein can restrict a number of viruses independently of the expression of other interferon-induced genes. Mx genes are therefore considered to be an important part of the innate antiviral immune response.

The amino acid sequence of MX1 has for reference UNIPROT P20591.

Antibodies able to specifically target MX1 (anti-MX1 antibodies) are well known in the art. Can for example be mentioned the monoclonal antibody LS-C784193 (commercialized by LifeSpan BioSciences), the polyclonal antibody PA522101 (commercialized by Invitrogen) or the monoclonal antibody ABIN6284316 (commercialized by Antibodies online).

Protein RTN4 (Reticulon-4), commonly known as a neurite outgrowth inhibitor (Nogo), is a surface protein expressed in neurons, which interacts with Brain-specific angiogenesis inhibitors (BAI) adhesion-GPCRs (G protein-coupled receptors) located in either neurons or glia via a glycan-mediated interface, and this interaction regulates the dendritic arborization, axonal elongation and formation of synapses in cultured human neurons.

The amino acid sequence of RTN4 has for reference UNIPROT Q9NQC3.

Antibodies able to specifically target RTN4 (anti-RTN4 antibodies) are well known in the art. Can for example be mentioned the polyclonal antibody LS-C753464 (commercialized by LifeSpan BioSciences), the monoclonal antibody ab250023 (commercialized by Abcam) or the polyclonal antibody ABIN3176379 (commercialized by Antibodies online).

Protein FASN (Fatty acid synthase) is a multi-enzyme protein that serves as the key regulator in lipid metabolism, especially fatty acid synthesis.

The amino acid sequence of FASN has for reference UNIPROT A0A0U1RQF0.

Antibodies able to specifically target FASN (anti-FASN antibodies) are well known in the art. Can for example be mentioned the monoclonal antibody 15973814 (commercialized by Fischer Scientific), the polyclonal antibody LS-B3636 (commercialized by LifeSpan BioSciences) or the polyclonal antibody GTX109833 (commercialized by GeneTex).

Protein PDIA4 (protein disulfide isomerase family A member 4) is a redoxdependent protein with both chaperone and oxidoreductase activities, and is originally discovered in the endoplasmic reticulum (ER) and participates in protein folding.

The amino acid sequence of PDIA4 has for reference UNIPROT P13667.

Antibodies able to specifically target PDIA4 (anti-PDIA4 antibodies) are well known in the art. Can for example be mentioned the polyclonal antibody LS-B3756 (commercialized by LifeSpan BioSciences), the monoclonal antibody CF503887 (commercialized by LifeSpan BioSciences) or the polyclonal antibody A305-266A (commercialized by Bethyl Laboratories).

A combination of antibodies for use according to the invention may comprise at least a fourth antibody, or an antigen binding fragment thereof, specifically binding to an antigen selected from the group consisting of SLC3A2, CKAP4, Annexin A2, GSTO1, Annexin A5, MX1, RTN4, FASN and PDIA4. Said antigen targeted by the said at least fourth antibody may in particular be different from the three antigens to which the first, second and third antibodies, or antigen-binding fragments thereof, bind to.

Accordingly, a combination of antibodies, or antigen-binding fragments thereof, for use according to the invention may comprise:
- at least a first antibody, or an antigen-binding fragment thereof,
- at least a second antibody, or an antigen-binding fragment thereof,
- at least a third antibody, or an antigen-binding fragment thereof, and
- at least a fourth antibody, or an antigen-binding fragment thereof,

each of these antibodies independently and specifically binding to an antigen selected from the group consisting of SLC3A2, CKAP4, Annexin A2, GSTO1, Annexin A5, MX1, RTN4, FASN and PDIA4,
each of the first, second, third and fourth antibodies binding to an antigen different from the one bound by the others.

In a particular embodiment, the combination of antibodies for use according to the invention is such that:
- the first antibody, or an antigen-binding fragment thereof, specifically binds to antigen GSTO1;
- the second antibody, or an antigen-binding fragment thereof, specifically binds to antigen Annexin A5;
- the third antibody, or an antigen-binding fragment thereof, specifically binds to antigen MX1; and
- the fourth antibody, or an antigen-binding fragment thereof, specifically binds to antigen RTN4.

A combination of antibodies for use according to the invention may consist in:
- a first monoclonal or polyclonal antibody, or an antigen-binding fragment thereof, which specifically binds to antigen GSTO1; and in particular a first monoclonal or polyclonal antibody which specifically binds to antigen GSTO1;
- a second monoclonal or polyclonal antibody, or an antigen-binding fragment thereof, which specifically binds to antigen Annexin A5; a second monoclonal or polyclonal antibody which specifically binds to antigen Annexin A5;
- a third monoclonal or polyclonal antibody, or an antigen-binding fragment thereof, which specifically binds to antigen MX1; and in particular a third monoclonal or polyclonal antibody which specifically binds to antigen MX1; and
- a fourth monoclonal or polyclonal antibody, or an antigen-binding fragment thereof, which specifically binds to antigen RTN4; and in particular a fourth monoclonal or polyclonal antibody which specifically binds to antigen RTN4.

An example of such a combination is illustrated in the examples of the present specification under the denomination XON5.

In a particular embodiment, the combination of antibodies for use according to the invention is such that:
- the first antibody, or an antigen-binding fragment thereof, specifically binds to antigen Annexin A2;
- the second antibody, or an antigen-binding fragment thereof, specifically binds to antigen SLC3A2;
- the third antibody, or an antigen-binding fragment thereof, specifically binds to antigen CKAP4; and
- the fourth antibody, or an antigen-binding fragment thereof, specifically binds to antigen PDIA4.

A combination of antibodies for use according to the invention may consist in:
- a first monoclonal or polyclonal antibody, or an antigen-binding fragment thereof, which specifically binds to antigen Annexin A2; and in particular a first monoclonal or polyclonal antibody which specifically binds to antigen Annexin A2;
- a second monoclonal or polyclonal antibody, or an antigen-binding fragment thereof, which specifically binds to antigen SLC3A2; a second monoclonal or polyclonal antibody which specifically binds to antigen SLC3A2;
- a third monoclonal or polyclonal antibody, or an antigen-binding fragment thereof, which specifically binds to antigen CKAP4; and in particular a third monoclonal or polyclonal antibody which specifically binds to antigen CKAP4; and
- a fourth monoclonal or polyclonal antibody, or an antigen-binding fragment thereof, which specifically binds to antigen PDIA4; and in particular a fourth monoclonal or polyclonal antibody which specifically binds to antigen PDIA4.

An example of such a combination is illustrated in the examples of the present specification under the denomination XON7.

In a particular embodiment, the combination of antibodies for use according to the invention is such that:
- the first antibody, or an antigen-binding fragment thereof, specifically binds to antigen SLC3A2;
- the second antibody, or an antigen-binding fragment thereof, specifically binds to antigen Annexin A5;
- the third antibody, or an antigen-binding fragment thereof, specifically binds to antigen RTN4;
- the fourth antibody, or an antigen-binding fragment thereof, specifically binds to antigen FASN; and
- a fifth antibody, or an antigen-binding fragment thereof, which specifically binds to antigen PDIA4.

A combination of antibodies for use according to the invention may consist in:
- a first monoclonal or polyclonal antibody, or an antigen-binding fragment thereof, which specifically binds to antigen SLC3A2;
- a second monoclonal or polyclonal antibody, or an antigen-binding fragment thereof, which specifically binds to antigen Annexin A5;
- a third monoclonal or polyclonal antibody, or an antigen-binding fragment thereof, which specifically binds to antigen RTN4; and in particular a third monoclonal or polyclonal antibody which specifically binds to antigen RTN4;
- a fourth monoclonal or polyclonal antibody, or an antigen-binding fragment thereof, which specifically binds to antigen FASN; and in particular a fourth monoclonal or polyclonal antibody which specifically binds to antigen FASN; and
- a fifth antibody, or an antigen-binding fragment thereof, which specifically binds to antigen PDIA4; and in particular a fifth antibody which specifically binds to antigen PDIA4.

An example of such a combination is illustrated in the examples of the present specification under the denomination XON9.

As indicated previously, in all of these embodiments, the antibodies of the combinations may, independently from the others, be devoid of at least one antigenic determinant selected from (i) N-glycolylneuraminic acid (Neu5Gc) and (ii) α-1,3-galactose, and may more particularly be devoid of the two antigenic determinants N-glycolylneuraminic acid (Neu5Gc) and α-1,3-galactose.

In a particular embodiment, in all of the above-mentioned embodiments, the antibodies of the combinations are devoid of at least one antigenic determinant selected from (i) N-glycolylneuraminic acid (Neu5Gc) and (ii) α-1,3-galactose, and are more particularly devoid of the two antigenic determinants N-glycolylneuraminic acid (Neu5Gc) and α-1,3-galactose.

The antibodies of a combination for use according to the invention may be of any origin, such as from a non-human mammal or synthetic. Such non-human mammal may be selected from the group consisting of rodents, such as mice, rats, guinea pigs and hamsters; lagomorphs, such as rabbits; ferrets; felines, such as cats; canines, such as dogs; goats; sheep; bovines, such as cows; swines, such as pigs and hogs; camelids; horses; and non-human primates.

The antibodies of a combination for use according to the invention are preferably from a pig, and in particular from a pig lacking at least one gene selected in a group comprising (i) a gene encoding a functional cytidine-5'-monophosphate N-acetyl neuraminic acid hydrolase (CMAH) and (ii) a gene encoding a functional α-(1,3)-galactosyltransferase, and more particularly lacking both (i) a gene encoding a functional cytidine-5'monophosphate N-acetyl neuraminic acid hydrolase (CMAH) and (ii) a gene encoding a functional α-(1,3)-galactosyltransferase.

The present invention also relates to a pharmaceutical composition for use in the treatment of a cancer in a human subject in need thereof comprising a combination of antibodies, or antigen-binding fragments thereof, as defined above, and a pharmaceutically acceptable carrier.

The composition for its use according to the invention may be in liquid form.

The composition for its use according to the invention may be in a solid form, which includes a lyophilized form.

The composition used according to the invention may be formulated according to standard methods such as those described in Remington: The Science and Practice of Pharmacy (Lippincott Williams & Wilkins; Twenty first Edition, 2005).

A composition for use according to the invention may further comprise at least one additional anticancer drug different from the antibodies of the combination of antibodies.

The terms *"combination of antibodies"* are also used herein to designate *"a combination of antibodies, or antigen-binding fragments thereof.*

The additional anticancer drug may for example be selected from the group consisting of monoclonal antibodies, in particular selected from the group consisting of anti-CD137, anti-CTLA4, anti-TIM-3, anti-B7-H3, anti-CD134, anti-CD154, anti-LAG-3, anti-CD227, anti-BTNA3, anti-CD39, anti-CD73, anti-CD115, anti-CD47, anti-SIRP alpha, anti-SIRP gamma, anti-CD28, anti-NCR, anti-NKp46, anti-NKp30, anti-NKp44, anti-NKG2D, anti-PD1, anti-PDL1 and anti-DNAM-1 monoclonal antibodies.

Dosages may range from 0.001 to 100 mg or more of the combination for use according to the invention as defined herein per kg of body weight (mg/kg) or greater, for example 0.1, 1.0, 10, or 50 mg/kg of body weight, with 1 to 20 mg/kg being preferred. The dosage and frequency of administration may be adapted as previously detailed.

Also, any injection of a combination for use according to the invention or composition for use according to the invention may be followed by any usual procedure to prevent and/or avoid anaphylactic reaction.

Besides, the injection of a combination or composition implemented according to the invention may be performed through a large peripheral access or, if possible, through a central catheter.

As is known in the art, adjustments for protein degradation, systemic versus localized delivery, as well as the age, body weight, general health, sex, diet, time of administration, possible allergy, drug interaction and the severity of the condition may be necessary, and is easily determined with routine experimentation by those skilled in the art.

Administration of the combination or composition for use of the invention may be done in a variety of ways, including, but not limited to, orally, subcutaneously, intravenously, parenterally, intranasally, intrarespiratory (such as nebulization or intratracheal spray), intraortically, intraocularly, rectally, vaginally, transdermally, topically (e.g., gels), intraperitoneally, intramuscularly, intrapulmonary or intrathecally.

Administration of the combination or composition of the invention may be done by following the Besredka method.

A combination or composition according to the invention may in particular be in a form suitable for administration by intravenous route.

### 3. Implementation of a combination of antibodies, or antigen-binding fragments thereof, or a composition, according to the invention

A combination of antibodies, or antigen-binding fragments thereof, or a composition comprising it, implemented according to the invention is used in a therapeutically effective amount.

As previously indicated, a combination of antibodies or composition thereof is for use in the treatment of a cancer in a human subject.

The cancer may in particular be selected from the group consisting of myeloma; melanoma; breast cancer; prostate cancer; colorectal cancer, in particular a colon cancer; lung cancer, in particular non small cell lung cancer; liver cancer, in particular an hepatocarcinoma; pancreas cancer; lymphoma, in particular B-cell or T-cell lymphoma, more particularly T-cell lymphoma; gastric cancer; head and neck cancer; ovarian cancer; sarcoma; leukemia, osteosarcoma; anal cancer; testicular cancer; uterus cancer; thyroid cancer; cancer of the central nervous system; gastrointestinal stromal cancer, epidermal carcinoma and oesophageal cancer.

In a particular embodiment, a combination for use according to the invention is such that:
- the first antibody, or an antigen-binding fragment thereof, specifically binds to antigen GSTO 1; and in particular the first antibody specifically binds to antigen GSTO1;
- the second antibody, or an antigen-binding fragment thereof, specifically binds to antigen Annexin A5; and in particular the second antibody specifically binds to antigen Annexin A5;
- the third antibody, or an antigen-binding fragment thereof, specifically binds to antigen MX1; and in particular the third antibody specifically binds to antigen MX1; and
- the fourth antibody, or an antigen-binding fragment thereof, specifically binds to antigen RTN4; and in particular the fourth antibody specifically binds to antigen RTN4;
with the cancer being selected from the group consisting of melanoma; myeloma; a liver cancer, in particular an hepatocarcinoma; breast cancer; prostate cancer; colorectal cancer, in particular a colon cancer; and lung cancer, in particular non small cell lung cancer, more particularly selected from the group consisting of melanoma; prostate cancer; myeloma; a liver cancer, in particular an hepatocarcinoma; colorectal cancer, in particular a colon cancer and lung cancer, in particular non small cell lung cancer; in particular selected from the group consisting of melanoma; prostate cancer; myeloma; a liver cancer, in particular an hepatocarcinoma and lung cancer, in particular non small cell lung cancer; and more particularly selected from the group consisting of melanoma; myeloma and a liver cancer, in particular an hepatocarcinoma.

In a particular embodiment, a combination for use according to the invention is such that:
- the first antibody, or an antigen-binding fragment thereof, specifically binds to antigen Annexin A2; and in particular the first antibody specifically binds to antigen Annexin A2;
- the second antibody, or an antigen-binding fragment thereof, specifically binds to antigen SLC3A2; and in particular the second antibody specifically binds to antigen SLC3A2;
- the third antibody, or an antigen-binding fragment thereof, specifically binds to antigen CKAP4; and in particular the third antibody specifically binds to antigen CKAP4; and
- the fourth antibody, or an antigen-binding fragment thereof, specifically binds to antigen PDIA4; and in particular the fourth antibody specifically binds to antigen PDIA4;

with the cancer being selected from the group consisting of myeloma; melanoma; breast cancer; prostate cancer; a liver cancer, in particular an hepatocarcinoma; colorectal cancer, in particular a colon cancer; lung cancer, in particular non small cell lung cancer; pancreas cancer; lymphoma, in particular B-cell or T-cell lymphoma, more particularly T-cell lymphoma; ovarian cancer; sarcoma; leukemia; osteosarcoma; testicular cancer; uterus cancer; thyroid cancer; cancer of the central nervous system; gastrointestinal stromal cancer and epidermal carcinoma;
more particularly selected from the group consisting of myeloma; melanoma; breast cancer; prostate cancer; a liver cancer, in particular an hepatocarcinoma; colorectal cancer, in particular a colon cancer; lung cancer, in particular non small cell lung cancer; pancreas cancer and lymphoma, in particular T-cell lymphoma;
even more particularly selected from the group consisting of myeloma; melanoma; breast cancer; a liver cancer, in particular an hepatocarcinoma; colorectal cancer, in particular a colon cancer; lung cancer, in particular non small cell lung cancer; pancreas cancer and lymphoma, in particular T-cell lymphoma;
more particularly selected from the group consisting of myeloma; melanoma; a liver cancer, in particular an hepatocarcinoma and colorectal cancer, in particular a colon cancer; and
in particular selected from the group consisting of myeloma; melanoma; and a liver cancer, in particular an hepatocarcinoma.

In a particular embodiment, a combination for use according to the invention is such that:
- the first antibody, or an antigen-binding fragment thereof, specifically binds to antigen SLC3A2; and in particular the first antibody specifically binds to antigen SLC3A2;
- the second antibody, or an antigen-binding fragment thereof, specifically binds to antigen Annexin A5; and in particular the second antibody specifically binds to antigen Annexin A5;
- the third antibody, or an antigen-binding fragment thereof, specifically binds to antigen RTN4; and in particular the third antibody specifically binds to antigen RTN4;
- the fourth antibody, or an antigen-binding fragment thereof, specifically binds to antigen FASN; and in particular the fourth antibody specifically binds to antigen FASN; and
- a fifth antibody, or an antigen-binding fragment thereof, which specifically binds to antigen PDIA4; and in particular a fifth antibody which specifically binds to antigen PDIA4;

with the cancer being selected from the group consisting of a liver cancer, in particular an hepatocarcinoma; myeloma; melanoma; colorectal cancer, in particular a colon cancer; lung cancer, in particular non small cell lung cancer; gastric cancer; head and neck cancer; anal cancer and oesophageal cancer,
in particular is selected from the group consisting of a liver cancer, in particular an hepatocarcinoma, myeloma; melanoma and a colorectal cancer, in particular a colon cancer;
and is more particularly a liver cancer, in particular an hepatocarcinoma.

The present disclosure is further illustrated, without in any way being limited to, by the Examples hereafter.

### EXAMPLES

### Example 1: In vitro efficacy of combinations of antibodies according to the invention on various human cancer cell lines

### Immunization of the animals

A protocol to obtain the specific CMAH and GAL double knockout non-human transgenic mammal is described in Lutz AL et al. (Xenotransplantation, 2013; 20 (1): 27-35) or in Conchon S. et al. (Xenotransplantation; special issue International Xenotransplantation Association IXA 2013, 2013, Vol. 20, Issue 5).

The combinations of pig antibodies XON5, XON7 and XON9 are obtained by immunization of GGTA1/CMAH KO pigs with cells genetically expressing on their surface:
- for XON5: GSTO1, Annexin 5, MX1 and RTN4.

XON5 is accordingly a combination comprising anti-GSTO1, anti-Annexin 5, anti-MX1 and anti-RTN4 antibodies obtained from a double knockout pig as defined above;
- for XON7: SLC3A2, CKAP4, Annexin A2 and PDIA4.

XON7 is accordingly a combination comprising anti- Annexin A2, anti-SLC3A2, anti- CKAP4 and anti-PDIA4 antibodies obtained from a double knockout pig as defined above; and
- for XON9: SLC3A2, Annexin 5, RTN4, FASN and PDIA4.

XON9 is accordingly a combination comprising anti-SLC3A2, anti-Annexin 5, anti-RTN4, anti-FASN and anti-PDIA4 antibodies obtained from a double knockout pig as defined above.

Immunization steps are followed as described in Lutz AL et al. (Xenotransplantation, 2013; 20 (1): 27-35) and in Conchon S. et al. (Xenotransplantation; special issue International Xenotransplantation Association IXA 2013, 2013, Vol. 20, Issue 5).

### Cross Binding Activity by FACS

100 000 cells were plated in conic 96-wells plate and incubated 30 min at 4°C with serial dilution of the corresponding purified IgG (maximal concentration 400 ug/mL). Then incubation with FITC-Prot A (1 :250) 30 min at 4°C and analyzed using a Flow Cytometer (BD FACSCanto^{™}; BD Biosciences, US).

### Tested cancer cell lines

Three different human tumor cell lines were tested:
- Hep-G2 (hepatocellular carcinoma),
- KMS-12-BM: (Myeloma), and
- SK-MEL-30 (Melanoma).

### Apoptosis assay on human PBMC

To assess apoptosis induction potential, all anti-cancer pAb were compared in an apoptosis assay.

Human PBMC or human cancer cell line were treated with increasing doses (10, 30, 100, 300 and 900µg/ml) of XON5, XON7 or XON9. Non-immune IgG were used as a negative control. After 3h culture (37°C, 5%CO2), apoptosis was monitored by flow cytometry by Annexin V and DAPI staining. The percentage of cells in Q3 (Annexin V+/DAPI- cells, early apoptosis) and in Q2 (Annexin V+/DAPI+, late apoptosis) were then added together to determine the percentage of apoptosis (n=3). (Figures 3 and 4).

### Comments

The cross-binding study of each XON5, XON7 and XON9 is presented in Figure 1. XON5 strongly targets the SK-MEL-30 cell line and weakly targets the Hep-G2 and KMS-12-BM cell lines. XON7 recognizes different antigens than XON5, and strongly targets the KMS-12-BM and SK-MEL-30 cell lines.

Interestingly, XON9, which shares 2 antigens recognized by XON7 and 2 antigens by XON5 and one specific to XON9, specifically and strongly recognizes the hepatocellular carcinoma cell line.

Then the cross-CDC activity of XON5, XON7 and XON9 was studied in several tumoral human cell lines. Results are presented in Figure 2.

All XON5, XON7 and XON9 were able to induce CDC against different cancer cell lines (Fig. 2 A to H).

XON5 was able to strongly lyse melanoma, non-small cell lung cancer, prostate cancer, hepatocellular carcinoma and breast cancer cell lines (between 60 to 100% of specific cytotoxicity at 800 µg/mL) and to a lesser extent colon and pancreatic adenocarcinoma cell lines (25% and 45% of specific cytotoxicity at 800 µg/mL). XON7 was able to strongly lyse myeloma, prostate cancer, breast cancer, hepatocellular carcinoma cell lines (between 80 to 100% of specific cytotoxicity at 800 µg/mL) and to a lesser extent colon and pancreatic adenocarcinoma, non-small cell lung cancer and melanoma cell lines (20% and 45% of specific cytotoxicity at 800 µg/mL ). XON9 was able to strongly lyse hepatocellular carcinoma cell line (with 80% of apoptotic cells at a concentration of 200 µg/mL) and to a lesser extent colon adenocarcinoma, pancreatic adenocarcinoma, non-small cell lung cancer, melanoma cell lines.

Apoptosis was studied in order to determine if this mechanism could be activated against the different human cell lines by anti-cancer pAb.

Results presented Figure 3 show that XON5, XON7 et XON9 are able to induce apoptosis of a melanoma and hepatocellular carcinoma cell line. XON5 has the highest apoptotic activity on melanoma cells (with 80% of apoptotic cells at a concentration of 300 µg/mL). XON9 has the highest apoptotic activity on hepatocellular carcinoma cells(with 80% of apoptotic cells at a concentration of 200 µg/mL) compared to apoptosis induced by a non-immune IgG (CT IgG).

In order to study the safety of XON5, XON7 and XON9, the apoptosis was studied on normal human PBMCs.

Figure 4 presents results showing no apoptosis of the normal PBMC by these combinations of antibodies.

### Example 2: In vivo therapeutic efficacy of XON5 or XON7 on human cancer models

### Model of human lung cancer / mammary carcinoma in mice

3.10E⁶ tumoral cells (A549, or NSCLC cell line) were injected into the sub-cutaneous left flank of 8 weeks old female NMRI-Nude according to the procedure described below.

Mice were injected subcutaneously in the left flank with 3.10⁶ tumoral cells in NaCl (100 µL total). Injection sites are continually examined for tumor growth until tumors reach 50-150 mm³. Mice are sorted into study groups, and then the compounds of interest are injected following the dosing schedule (35 mg/kg twice a week). Tumors were measured twice a week using a caliper. The end of the study was marked when tumors reached 2000 mm³ or if the tumor appeared necrotic (more than 50% of the surface).

3.10E⁶ tumoral cells MDA-MB-231 were injected into the mammary gland of 8 weeks old female NMRI-Nude according to the procedure described below.

Mice were injected in the mammary gland with 3.10⁶ tumoral cells in NaCl (50 µL total). Injection sites are continually examined for tumor growth until tumors reach 50-150 mm³. Mice are sorted into study groups, and then the compounds of interest are injected following the dosing schedule (35 mg/kg twice a week). Tumors were measured twice a week using a caliper. The end of the study was marked when tumors reached 2000 mm³ or if the tumor appeared necrotic (more than 50% of the surface).

### Comments:

The potential therapeutic efficacy of antibody combinations XON5 and XON7 was studied on different xenograft human cancer models in mice.

Figure 5 presents *in vivo* results of the median tumor volume after mice inoculation with A549 cell line and after treatment or not with XON5 or XON7 every week during 3 weeks. Both anti-cancer antibody combinations tested decreased significantly the tumor volume compared to the untreated animals showing the therapeutic potential on human lung cancer of XON5 and XON7.

The tumor volume of human breast cancer was followed during 3 weeks after cell inoculation and treatment with XON5 or XON7. Results are presented Figure 6 and show that both combinations of antibodies could present a therapeutic potential on human breast cancer.

A tissue Cross Reactivity was performed on normal and cancer tissues from human patients with XON5, XON7, or XON9 (see Table 1). These TMA (Charles River Discovery Research Services Germany) gather ten cancer tissues associated with their normal counterpart (brain, muscle, liver, kidney, breast, pancreas, colon, lung, skin, uterus).

After quenching endogenous peroxydase with H₂0₂ 3%, tissue microarrays were incubated with 5ug/µL of each antibody combinations. A goat anti-pig HRP was used as secondary antibody and then colored with ImmPACT^{™} VIP HRP substrate which produce intense purple reaction product.

The results obtained showed absolutely no cross-reactivity of XON5 and XON7 with normal non-cancerous tissues while cross-reactivity was observed with most corresponding cancerous tissues, indicating the specificity of the polyclonal antibodies considered in the present application against tumor tissues and suggesting a negative off target effect, and thus a good safety.

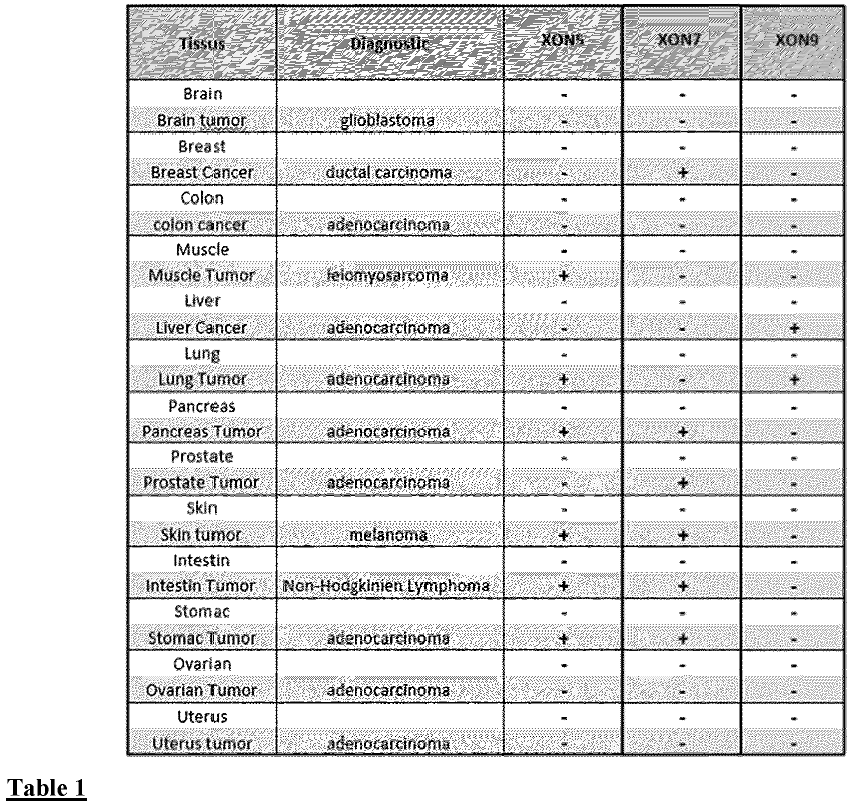

### Model of prostate cancer in mice

2.5 10E⁵ tumoral cells (LNCaP cell line) were injected into the sub-cutaneous left flank of 7 weeks old female NMRI-Nude mice according to the procedure described below.

Mice were injected subcutaneously in the left flank with 2.5 10E⁵ tumoral cells in NaCl (100 µL total) at Day 0. Mice are sorted into study groups, and then the compounds of interest are intraperitoneally injected following the dosing schedule (35 mg/kg twice a week) from D0 for a total duration of 28 days. Tumors were measured twice a week using a caliper. The end of the study was marked when tumors reached 1000 mm³ or if the tumor appeared necrotic (more than 50% of the surface).

The different groups were as follows: a control group (no treatment - n=10), a group administered with XON7 (Group "XON7" - n=10) and a group administered with XON5 (Group "XON5" - n=10).

### Comments:

Figure 7 presents *in vivo* results of the median tumor volume after mice inoculation as mentioned above and the effect of XON7 and XON5 on a prostate cancer cell line. Mice from the control group displayed a slow increase of the tumor size from Day 0, with median tumor size reaching 200 mm³ from Day 20 and reaching 400mm³ at the end of the protocol (Day 28). Mice from the treated groups (XON5 and XON7) displayed significantly slower tumor growth (One Way Anova - *** p<0,001) staying under 100mm³ at the end of the protocol (Day 28). Combination of antibodies according to the invention accordingly leads to a significant decrease of a prostate tumor growth.

### Model of colon adenocarcinoma in mice

2.5 10E⁵ human colon adenocarcinoma cells (HCT-116 cell line) were injected into the sub-cutaneous left flank of 7 weeks old female NMRI-Nude mice according to the procedure described below.

Mice were injected subcutaneously in the left flank with 2.5 10E⁵ tumoral cells in NaCl (100 uL total) at Day 0. Mice are sorted into study groups, and then the compounds of interest are intraperitoneally injected following the dosing schedule (35 mg/kg twice a week) from D0 for a total duration of 28 days. Tumors were measured twice a week using a caliper. The end of the study was marked when tumors reached 3000 mm³ or if the tumor appeared necrotic (more than 50% of the surface). From Day 28, survival mice are observed but no treatment are administered until the end of the protocol on Day 38.

The different groups were as follows: a control group (no treatment - n=10) and a group administered with XON7 (Group "XON7" - n=10).

### Comments:

Figure 8 presents *in vivo* results of the median tumor volume after mice inoculation as mentioned above and the effect of XON7 on a colon adenocarcinoma cell line. Mice from the control group displayed a rapid increase of the tumor size from Day 18, with median tumor size reaching 1000mm³ at the end of the protocol (Day 38). Mice from the treated group (XON7) displayed slower tumor growth staying around 500mm³ at the end of the protocol (Day 38). A combination of antibodies according to the invention accordingly leads to a significant decrease of a colon adenocarcinoma growth.

### Model of T Cell lymphoma in mice

2.5 10E⁵ T Cell lymphoma cells were injected into the sub-cutaneous left flank of 7 weeks old female NMRI-Nude mice according to the procedure described below.

Mice were injected subcutaneously in the left flank with 2.5 10E⁵ tumoral cells in NaCl (100 uL total) at Day 0. Mice are sorted into study groups, and then the compounds of interest are intraperitoneally injected following the dosing schedule (35 mg/kg twice a week) from D0 for a total duration of 28 days. Tumors were measured twice a week using a caliper. The end of the study was marked when tumors reached 3000 mm³ or if the tumor appeared necrotic (more than 50% of the surface).

The different groups were as follows: a control group (no treatment - n=10) and a group administered with XON7 (Group "XON7" - n=10).

### Comments:

Figure 9 presents *in vivo* results of the median tumor volume after mice inoculation as mentioned above and the effect of XON7 on a T Cell lymphoma. Mice from the control group displayed a slow increase of the tumor size from Day 0, with median tumor size reaching 100mm³ from Day 20 and reaching 180mm³ at the end of the protocol (Day 28). Mice from the treated group (XON7) displayed significantly slower (T test - ** p<0,01, * p<0,05) tumor growth staying under 100mm³ at the end of the protocol (Day 28). A combination of antibodies according to the invention obtained by immunization with XON7 accordingly lead to a significant decrease of a T Cell lymphoma growth.

### Example 3: In vitro comparative efficacy of various monoclonal antibodies, polyclonal antibodies, combinations thereof and XON5, XON7 and XON9 on various human cancer cell lines

### Antibodies and combinations of antibodies

The antibodies and combinations of antibodies implemented in these examples are as follow:
- an anti-SLC3A2 monoclonal antibody synthetically prepared on the basis of the teachings of WO2017214458;
- an anti-Annexin A2 monoclonal antibody synthetically prepared on the basis of the teachings of WO2018/021972;
- an anti-CKAP4 polyclonal antibody (i.e. a group of antibodies targeting different epitopes of CKAP4) synthetically obtained by immunization of rabbits using the peptides described in WO2019065747;
- an anti-GSTO1 polyclonal antibody (16310345 commercialized by Fischer Scientific);
- an anti-Annexin A5 polyclonal antibody (15725131 commercialized by Fischer Scientific);
- an anti-MX1 polyclonal antibody (13229538 commercialized by Fischer Scientific);
- an anti-RTN4 polyclonal antibody (ABIN3176379 commercialized by Antibodies-online);
- an anti-FASN polyclonal antibody (15973814 commercialized by Fischer Scientific);
- a combination ("combo") of the anti-Annexin A2 monoclonal antibody, the anti-SLC3A2 monoclonal antibody and the anti-CKAP4 polyclonal antibody;
- a combination ("combo A") of the anti-GSTO1 polyclonal antibody, the anti-Annexin A5 polyclonal antibody, the anti-MX1 polyclonal antibody and of the anti-RTN4 polyclonal antibody;
- a combination ("combo C") of the anti-SLC3A2 monoclonal antibody, the anti-Annexin A5 polyclonal antibody, the anti-FASN polyclonal antibody and the anti-RTN4 polyclonal antibody;
   and
- XON5, XON7 or XON9.

### Tested cancer cell lines

Four different human tumor cell lines were tested:
- SK-MEL-30 (Melanoma) (Figures 10 and 14);
- Capan1 (pancreatic adenocarcinoma cell line) (Figure 11);
- KMS-12-BM: (Myeloma) (Figure 12); and
- Hep-G2 (hepatocellular carcinoma) (Figures 13 and 15).

### Cross Binding Activity by FACS

300 000 cells were plated in conic 96-wells plate and incubated 30 min at 4°C with a dilution of the corresponding monoclonal antibody, polyclonal antibodies or XON5, XON7 or XON9 combination of antibodies according to the invention. After washes, a secondary anti-protein A antibody Alexa Fluor 488 conjugated was added in each condition and incubated 30 min at 4°C. Mean Fluorescence intensity was then analyzed using a NucleoCounter^{®} NC-3000^{™} Advanced Image Cytometer (Chemometec, Denmark).

### Comments

The comparative binding study of each single-target antibody as defined above, as well as specific combinations thereof "combos" defined above and XON5, XON9 or XON7 are presented in Figures 10 to 15 depending on the cancer cell line, the combination of antibodies and the "XON" (i.e. XON5, XON7 or XON9) tested.

In all these Figures, the combinations of single-target antibodies according to the invention detailed above ("combo" and "XON") more strongly recognize the targeted cancer cell line than what could have been expected when considering the binding results obtained with the monotherapies. Indeed, one skilled in the art would have expected either an addition of the single target effect of each component of the combination, or a reduction of the total effect due to steric hindrance of the antibodies.

Interestingly, the "combo C" combination, although having one common antibody to the "combo" combination, showed a stronger recognition of the Hep-G2 cell line, thus demonstrating that the recognition is not related to the stronger recognition of one antibody but to the combination of all antibodies. The combination is indeed a different product than the antibody alone.

### Apoptosis assay

To assess apoptosis induction potential, all these antibodies and combinations of antibodies were compared in an apoptosis assay.

Human cancer cell lines KMS-12-BM (Figure 19A) and SK-MEL-30 (Figure 19B) were treated with the above indicated monoclonal antibodies, polyclonal antibodies, or combinations of antibodies according to the invention (i.e. "combo" as defined above or combination XON7) at the above indicated doses. After 3 hours of incubation at 37°C, Annexin V-CF488A conjugate and Hoechst 33342 (final concentration: 10 µg/mL) were added and incubated for 15 min at 37°C. After washes, cell pellets were resuspended in 100 µL Annexin V binding buffer supplemented with 10 µg/mL PI. Apoptotic cells were analyzed using NucleoCounter^{®} NC-3000^{™} Advanced Image Cytometer (Chemometec, Denmark).

### Comments

While monoclonal or polyclonal antibodies tested alone show no sign of apoptosis of the cancer cells, the "combo" and XON7 show apoptosis activities against the two cancer cell lines. More particularly, while the "combo" show about 10% apoptotic cells with the KMS-12-BM cell line, XON7 percentage is above 60%. Concerning the SK-MEL-30 cell line, a similar apoptosis activity is observed between the "combo" and XON7.

### Example 4: In vitro and in vivo therapeutic efficacy of XON7 on human cancer models

### Antibodies and combinations of antibodies

The antibodies and combinations of antibodies tested in these examples are the same as in Example 3, i.e.:
- an anti-Annexin A2 monoclonal synthetically prepared on the basis of the teachings of WO2018/021972 ;
- an anti-SLCA32 monoclonal antibody synthetically prepared on the basis of the teachings of WO2017214458;
- an anti-CKAP4 polyclonal antibody (i.e. a group of antibodies targeting different epitopes of CKAP4) synthetically obtained by immunization of rabbits using the peptides described in WO2019065747;
- a combination ("combo") of the anti-Annexin A2 monoclonal antibody, the anti-SLC3A2 monoclonal antibody and the anti-CKAP4 polyclonal antibody; and
- XON7, as defined above in Example 1, in presence of rabbit complement (dilution 1/3 final).

Three different human tumor cell lines were tested *in vitro:*
- HCT-116 (colon adenocarcinoma) (Figure 16);
- Hep-G2 (hepatocellular carcinoma) (Figure 17); and
- KMS-12-BM: (Myeloma) (Figure 18).

Cell line KMS-12-BM (Myeloma) was also tested *in vivo.*

### In vitro Complement Dependent Cytotoxicity (CDC) assay

To assess cytotoxicity potential, all these antibodies and combinations of antibodies were compared in an apoptosis assay.

Human cancer cell lines HCT-116 (Figure 16), Hep-G2 (Figure 17) and KMS-12-BM (Figure 18) were treated with the above indicated monoclonal antibodies, polyclonal antibody, or combinations of antibodies according to the invention (i.e. "combo" as defined above or combination XON7) at the above indicated doses. After 30 min of incubation at 37°C, cell viability was done using NucleoCounter^{®} NC-3000^{™} Advanced Image Cytometer (Chemometec, Denmark) after adding AO/DAPI.

### Comments

On the three cancer cell lines, only the anti-SLC3A2 monoclonal antibody show a significant specific cytotoxicity (respectively about 45%, about 30% and about 35%), with the anti-CKAP4 polyclonal antibody showing respectively specific cytotoxicity of slightly above 0%, about 8% and 0%. No cytotoxicity is observed with the anti-Annexin A2 monoclonal antibody.

On the contrary, the combinations of antibodies according to the invention ("combo" and XON7) specific cytotoxicity percentages are significantly and unexpectedly higher.

More particularly, on the colon adenocarcinoma cell line, this percentage is similar for the "combo" and XON7, with said percentage being close to 70% for the "combo" and of about 80% with XON7. Similarly, on the colon the hepatocellular carcinoma cell line, this percentage is close to 50% for the "combo" and of about 70% with XON7. Finally, on the myeloma cell line, this percentage is also close to 50% for the "combo" and of about 70% with XON7.

### In vivo Myeloma model

Mice were injected subcutaneously in the left flank with 3.10⁶ KMS-12-BM tumoral cells in NaCl (100 µL total) at day 0. Treatment is initiated from the beginning of tumor growth and occurs twice a week for a total duration of 28 days. Treatment consists of the intraperitoneal injection of the anti-Annexin A2 monoclonal antibody (n=10); the anti-SLC3A2 monoclonal antibody (n=10); the anti-CKAP4 polyclonal antibody (n=10); the "Combo" (i.e. a combination comprising as actives the anti-Annexin A2 antibody + the anti-SLC3A2 antibody + the anti-CKAP4 antibody (n=10); XON7 (n=10); no treatment for Group "Negative control" (n=10).

### Comments

Mice From groups receiving the anti-Annexin A2 monoclonal antibody, the anti-SLC3A2 monoclonal antibody and the anti-CKAP4 polyclonal antibody in monotherapy induces respectively 75%, 71% and 43% of tumor growth reduction. On the contrary, the combination of the 3 antibodies ("combo") almost abolishes the tumor growth, similar to the treatment with XON7 (82% and 85% respectively) (see Figure 20).

### Example 5: Cancer cross-reactivity of XON5, XON7 and XON9 on PDX Tumor Tissue micro array

OncotestTM PDX tumor TMA slides were provided by Charles River Discovery Research Services Germany. Briefly after deparaffinization, antigen retrieval with citrate buffer and peroxidase quenching, slides were incubated with XON5, XON7 and XON9 at 5 µg/mL for all overnight at 4°C. An anti-pig IgG-HRP was used as secondary antibody and incubated during 2h at room temperature. Then, revelation/staining was made using ImmPACT^{™} VIP Peroxidase substrate. (LSBio).

The different results obtained are represented in the following Tables:

**Table 2**

| XON5 staining of human cancer biopsies | Number of stained patient's biopsies/number of tested biopsies | Mean % of stained area in positive biopsies |
|---|---|---|
| Asian colon cancer | 1/4 | 3,44 % |
| Colon cancer | 56/63 | 22,18 % +/- 2,75% |
| Non-small cell lung cancer | 39/41 | 47,74 % +/- 4,94% |
| Lung cancer of Asian ethnicity | 5/5 | 45,32 % +/- 5,65% |
| Lung cancer | 2/2 | 29,89 % +/- 19,95% |
| Melanoma | 27/27 | 94,89% +/- 5% |

**Table 3**

| XON7 staining of human cancer biopsies | Number of stained patient's biopsies/number of tested biopsies | Mean % of stained area in positive biopsies |
|---|---|---|
| Asian colon cancer | 4/4 | 86,83% +/- 4,12% |
| Colon cancer | 45/63 | 19,52% +/- 2,57% |
| prostate cancer | 1/1 | 19,23% |
| breast cancer HER2 | 4/7 | 6,75% +/- 3,6% |
| Breast cancer triple neg | 10/12 | 14,49% +/- 4% |
| Ovarian cancer | 13/16 | 10,42% +/- 2,6% |
| Testicular cancer | 1/2 | 18,33 % |
| uterus cancer | 1/1 | 24,3 % |
| Thyroid cancer | 3/4 | 43,38% +/- 24,15 % |
| Xenograph of asian ethnicity | 1/1 | 75,3 % |
| Non-small cell lung cancer epidermoid | 29/30 | 69,7 % +/- 3,4 % |
| Non-small cell lung cancer large cell | 9/10 | 70,8 % +/- 7,1 % |
| Small cell lung cancer | 9/9 | 84,5% +/- 6,6% |
| pleura-mesothelioma | 9/9 | 85,2% +/- 5,1% |
| Cancer of the central nervous system | 13/13 | 37,01% +/- 5,5% |
| Gastrointestinal stromal cancer | 2/2 | 64,65% +/- 25,1% |
| epidermal carcinoma | 1/1 | 48,15% |
| sarcoma of Asian ethnicity | 1/1 | 58,3% |
| Osteosarcoma | 3/4 | 58,41% +/- 17,7% |
| Soft tissue sarcoma | 10/10 | 40,15% +/- 9,2% |
| Leukemia | 1/1 | 84,85% |
| Acute lymphoblastic leukemia | 1/1 | 83,14% |
| Acute myeloid leukemia | 10/10 | 79,36% +/- 5,4% |
| Diffuse large B cell lymphoma | 7/7 | 70,5% +/- 9,7% |
| Pancreas cancer | 35/48 | 24,2% +/- 3,1% |

**Table 4**

| XON9 staining of human cancer biopsies | Number of stained patient's biopsies/number of tested biopsies | Mean % of stained area in positive biopsies |
|---|---|---|
| Asian colon cancer | 3/4 | 4,14% +/- 1,14% |
| Colon cancer | 53/63 | 18,15% +/- 2,9% |
| Cancer anal | 2/2 | 1,2% +/- 0,05% |
| Gastric cancer of asian ethnicity | 16/27 | 45,32% +/- 4,07% |
| head and neck cancer | 7/10 | 9,08% +/- 2,76% |
| Oesophageal cancer | 27/27 | 2,42% |
| Liver cancer, cholangiocellular carcinoma | 8/9 | 8,31% +/- 2,08% |
| Liver cancer, hepatocellular carcinoma | 2/2 | 6,08% +/- 2,74 % |
| Non-small cell lung cancer, epidermoid | 20/30 | 16,85% +/- 4,52 % |
| Non-small cell lung cancer, large cell | 7/10 | 10,71% +/- 3,89% |
| Small cell lung cancer | 9/9 | 16,26% +/- 5,83% |
| Pleuramesothelioma | 6/9 | 12,69% +/- 9,71% |

Tables include all patient tumours analyzed by TMAs. Summarized are the number of biopsies recognized by the combinations of antibodies, as well as the average percentage of the area of marked tissues. All antibody combinations XONs display a cross reactivity with several cancers, with certain cancers recognized specifically for each XON or shared by several XONs.

## Claims

1. A combination of antibodies, or antigen-binding fragments thereof, for use in the treatment of a cancer in a human subject in need thereof, the combination comprising:
- at least a first antibody, or an antigen-binding fragment thereof,
- at least a second antibody, or an antigen-binding fragment thereof, and
- at least a third antibody, or an antigen-binding fragment thereof,
each of these antibodies independently and specifically binding to an antigen selected from the group consisting of SLC3A2, CKAP4, Annexin A2, GSTO1, Annexin A5, MX1, RTN4, FASN and PDIA4,
wherein the first, second and third antibodies, or antigen-binding fragments thereof, each bind to a different antigen selected from the group consisting of SLC3A2, CKAP4, Annexin A2, GSTO1, Annexin A5, MX1, RTN4, FASN and PDIA4.

2. The combination for use according to claim 1, comprising at least a fourth antibody, or an antigen binding fragment thereof, specifically binding to an antigen selected from the group consisting of SLC3A2, CKAP4, Annexin A2, GSTO1, Annexin A5, MX1, RTN4, FASN and PDIA4,
said antigen being in particular different from the three antigens to which the first, second and third antibodies, or antigen-binding fragments thereof, bind to.

3. The combination for use according to claim 1 or 2, wherein the antigen-binding fragments of the antibodies present in the combination are independently Fv, Fab, F(ab')2, Fab', dsFv, (dsFv)2, scFv, sc(Fv)2, or diabodies.

4. The combination for use according to any one of claims 1 to 3, wherein the cancer is selected from the group consisting of myeloma; melanoma; breast cancer; prostate cancer; colorectal cancer, in particular a colon cancer; lung cancer, in particular non small cell lung cancer; liver cancer, in particular an hepatocarcinoma; pancreas cancer; lymphoma, in particular B-cell or T-cell lymphoma, more particularly T-cell lymphoma; gastric cancer; head and neck cancer; ovarian cancer; sarcoma; leukemia, osteosarcoma; anal cancer; testicular cancer; uterus cancer; thyroid cancer; cancer of the central nervous system; gastrointestinal stromal cancer, epidermal carcinoma and oesophageal cancer.

5. The combination for use according to any one of claims 1 to 4, wherein:
- the first antibody, or an antigen-binding fragment thereof, specifically binds to antigen GSTO1;
- the second antibody, or an antigen-binding fragment thereof, specifically binds to antigen Annexin A5;
- the third antibody, or an antigen-binding fragment thereof, specifically binds to antigen MX1; and
- the fourth antibody, or an antigen-binding fragment thereof, specifically binds to antigen RTN4;
- the cancer being selected from the group consisting of melanoma; myeloma; a liver cancer, in particular an hepatocarcinoma; breast cancer; prostate cancer; colorectal cancer, in particular a colon cancer; and lung cancer, in particular non small cell lung cancer,
more particularly selected from the group consisting of melanoma; prostate cancer; myeloma; a liver cancer, in particular an hepatocarcinoma; colorectal cancer, in particular a colon cancer and lung cancer, in particular non small cell lung cancer;
in particular selected from the group consisting of melanoma; prostate cancer; myeloma; a liver cancer, in particular an hepatocarcinoma and lung cancer, in particular non small cell lung cancer; and
more particularly selected from the group consisting of melanoma; myeloma and a liver cancer, in particular an hepatocarcinoma.

6. The combination for use according to any one of claims 1 to 4, wherein:
- the first antibody, or an antigen-binding fragment thereof, specifically binds to antigen Annexin A2;
- the second antibody, or an antigen-binding fragment thereof, specifically binds to antigen SLC3A2;
- the third antibody, or an antigen-binding fragment thereof, specifically binds to antigen CKAP4; and
- the fourth antibody, or an antigen-binding fragment thereof, specifically binds to antigen PDIA4;
- the cancer being selected from the group consisting of myeloma; melanoma; breast cancer; prostate cancer; a liver cancer, in particular an hepatocarcinoma; colorectal cancer, in particular a colon cancer; lung cancer, in particular non small cell lung cancer; pancreas cancer; lymphoma, in particular B-cell or T-cell lymphoma, more particularly T-cell lymphoma; ovarian cancer; sarcoma; leukemia; osteosarcoma; testicular cancer; uterus cancer; thyroid cancer; cancer of the central nervous system; gastrointestinal stromal cancer and epidermal carcinoma;
more particularly selected from the group consisting of myeloma; melanoma; breast cancer; prostate cancer; a liver cancer, in particular an hepatocarcinoma; colorectal cancer, in particular a colon cancer; lung cancer, in particular non small cell lung cancer; pancreas cancer and lymphoma, in particular T-cell lymphoma;
even more particularly selected from the group consisting of myeloma; melanoma; breast cancer; a liver cancer, in particular an hepatocarcinoma; colorectal cancer, in particular a colon cancer; lung cancer, in particular non small cell lung cancer; pancreas cancer and lymphoma, in particular T-cell lymphoma;
more particularly selected from the group consisting of myeloma; melanoma; a liver cancer, in particular an hepatocarcinoma and colorectal cancer, in particular a colon cancer; and
in particular selected from the group consisting of myeloma; melanoma; and a liver cancer, in particular an hepatocarcinoma.

7. The combination for use according to any one of claims 1 to 4, wherein:
- the first antibody, or an antigen-binding fragment thereof, specifically binds to antigen SLC3A2;
- the second antibody, or an antigen-binding fragment thereof, specifically binds to antigen Annexin A5;
- the third antibody, or an antigen-binding fragment thereof, specifically binds to antigen RTN4; and
- the fourth antibody, or an antigen-binding fragment thereof, specifically binds to antigen FASN;
wherein the combination further comprises a fifth antibody, or an antigen-binding fragment thereof, which specifically binds to antigen PDIA4;
- the cancer being selected from the group consisting of a liver cancer, in particular an hepatocarcinoma; myeloma; melanoma; colorectal cancer, in particular a colon cancer; lung cancer, in particular non small cell lung cancer; gastric cancer; head and neck cancer; anal cancer and oesophageal cancer,
in particular is selected from the group consisting of a liver cancer, in particular an hepatocarcinoma, myeloma; melanoma and a colorectal cancer, in particular a colon cancer;
and is more particularly a liver cancer, in particular an hepatocarcinoma.

8. The combination for use according to any one of claims 1 to 7, wherein at least one of the antibodies of the combination for use, and in particular all the antibodies of the combination for use, is/are devoid of at least one antigenic determinant selected from (i) N-glycolylneuraminic acid (Neu5Gc) and (ii) α-1,3-galactose, and in particular is/are devoid of the two antigenic determinants N-glycolylneuraminic acid (Neu5Gc) and α-1,3-galactose.

9. A pharmaceutical composition for use in the treatment of a cancer in a human subject in need thereof comprising a combination of antibodies, or antigen-binding fragments thereof, as defined in anyone of claims 1 to 3 and 5 to 8, and a pharmaceutically acceptable carrier.

10. The pharmaceutical composition for use according to claim 9, wherein the cancer is selected from the group consisting of myeloma; melanoma; breast cancer; prostate cancer; colorectal cancer, in particular a colon cancer; lung cancer, in particular non small cell lung cancer; liver cancer, in particular an hepatocarcinoma; pancreas cancer; lymphoma, in particular B-cell or T-cell lymphoma, more particularly T-cell lymphoma; gastric cancer; head and neck cancer; ovarian cancer; sarcoma; leukemia, osteosarcoma; anal cancer; testicular cancer; uterus cancer; thyroid cancer; cancer of the central nervous system; gastrointestinal stromal cancer, epidermal carcinoma and oesophageal cancer.

11. The pharmaceutical composition for use according to claim 9 or 10, wherein the composition further comprises at least one additional anticancer drug different from the antibodies of the combination of antibodies as defined in anyone of claims 1 to 3 and 5 to 8.

12. The polyclonal antibody composition for its use according to claim 11, wherein the at least one additional anticancer drug is selected from the group consisting of monoclonal antibodies, in particular selected from the group consisting of anti-CD137, anti-CTLA4, anti-TIM-3, anti-B7-H3, anti-CD134, anti-CD154, anti-LAG-3, anti-CD227, anti-BTNA3, anti-CD39, anti-CD73, anti-CD115, anti-CD47, anti-SIRP alpha, anti-SIRP gamma, anti-CD28, anti-NCR, anti-NKp46, anti-NKp30, anti-NKp44, anti-NKG2D, anti-PD1, anti-PDL1 and anti-DNAM-1 monoclonal antibodies.
